# EUROPEAN PATENT APPLICATION

(11) **EP 4 538 269 A1**
(43) Date of publication of application: **16.04.2025**
(21) Application number: 23819112.6
(22) Date of filing: 06.06.2023
(51) Int. Cl.: C07D 403/12, C07D 417/14, C07D 401/12, C07D 213/82, A61K 31/455, A61P 35/00

(54) **SUBSTITUTED PYRIDAZINE-3-CARBOXAMIDE COMPOUND SERVING AS TYK2 INHIBITOR**

(30) Priority: 07.06.2022 CN 202210638601; 13.04.2023 CN 202310398880
(71) Applicant: Guangzhou Fermion Technology Co., Ltd., Guangzhou, Guangdong 510005 (CN)
(72) Inventor: DENG, Daiguo, Guangzhou, Guangdong 510005 (CN); ZHONG, Wenhe, Guangzhou, Guangdong 510005 (CN); LU, Jielian, Guangzhou, Guangdong 510005 (CN); LEI, Zengrong, Guangzhou, Guangdong 510005 (CN)
(74) Representative: Mewburn Ellis LLP
(86) International application number: PCT/CN2023/098585
(87) International publication number: WO 2023/236947

(57) **Abstract**

The present invention provides a pyridazine-3-carboxamide compound represented by general formula (I), or a pharmaceutically acceptable salt, enantiomer, diastereomer, racemate, solvate, hydrate, polymorph, prodrug or isotopic variant thereof. The present invention further provides a pharmaceutical composition including the compound, a preparation method therefor and a use thereof in treating or preventing a TYK2 kinase-mediated disease.

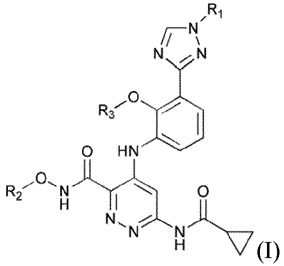

## Description

### FIELD OF THE INVENTION

The present invention relates to the field of medicinal chemistry, and in particular to a substituted pyridazine-3-carboxamide compound, a composition including the same, a preparation method therefor and a use thereof as a TYK2 inhibitor.

### BACKGROUND

The Janus kinase (JAK) family is made of intracellular non-receptor tyrosine kinases that mediate the signaling and activation of various cytokines. JAK gain-of-function expressions or mutations are associated with many autoimmune diseases, inflammation conditions and cancers. This family includes JAK1, JAK2, JAK3 and TYK2, among which JAK1, JAK2 and TYK2 are widely present in various tissues and cells of the human body, and JAK3 is mainly present in bone marrow cells, thymus cells, NK cells and activated B cells and T cells.

A JAK-mediated signaling pathway includes three key parts: a cytokine receptor on a cell surface, a JAK, and a downstream protein. Cytokines such as various interferons (IFNs) and interleukins (ILs) bind to the cytokine receptor on the cell surface, bringing into proximity the JAK that binds to an intracellular domain of the receptor. Then, a tyrosine residue of the JAK is phosphorylated, increasing the activity of the kinase domain. Next, the activated JAK phosphorylates a tyrosine residue of the receptor, creating a binding site for a protein having an SH2 domain. STAT (signal transducer and activator of transcription) binds to the phosphorylated tyrosine on the receptor through its SH2 domain, and is phosphorylated by the JAK and produces a phosphorylated STAT dimer, which then translocates to the nucleus to induce transcription of a target gene. In addition, other proteins having SH2 domains can also bind to the activated JAK, thereby cross-linking with other signaling pathways, such as PI3K/AKT, MAPK/ERK, etc.

TYK2 is a non-receptor tyrosine kinase that mediates immune signals. TYK2 mainly regulates signaling pathways driven by IL-23, IL-12 and type I interferon (IFNα), and is used as an IL-12, IL-23 and/or IFNa regulator by inhibiting TYK2-mediated signal transduction. TYK2 plays an important role in transmitting inflammatory and immune response signals, and is involved in pathophysiological processes of a variety of immune-related diseases, such as psoriasis (PS), rheumatoid arthritis (RA), systemic lupus erythematosus (SLE), inflammatory bowel disease (IBD), etc. TYK2 does not mediate cytokine responses driven by other kinases (such as IL-6, hematopoietic growth factor and IL-2). Therefore, TYK2 inhibitors can avoid adverse reactions associated with currently marketed JAK inhibitors by not acting on other subtypes.

Common small molecule JAK inhibitors are active site-directed inhibitors that bind to adenosine triphosphate (ATP) sites of the catalytic domains (JH1) of JAK proteins. Due to the high homology of the ATP sites of JAK family kinases and the similarity to the ATP-binding regions of the human kinase group, there generally exists an issue of low selectivity.

Studies have shown that the pseudokinase domain (JH2) in the JAK family, which exhibits significant catalytic activity, can provide an ideal allosteric site for the discovery of TYK2 selective inhibitors. Compound BMS-986165 is a currently known compound that can selectively bind to the JH2 of TYK2 and inhibit TYK2 kinase functions through an allosteric effect.

There is currently a need for drugs with improved activity in selectively inhibiting TYK2 by binding to JH2, thereby providing therapeutic benefits in the treatment of diseases.

### SUMMARY

In view of the foregoing, the present invention provides a substituted pyridazine-3-carboxamide compound, which has excellent activity for selective inhibition of TYK2 and can treat a variety of TYK2-mediated diseases.

The present invention provides a compound represented by general formula (I), or a pharmaceutically acceptable salt, enantiomer, diastereomer, racemate, solvate, hydrate, polymorph, prodrug or isotopic variant thereof, and a mixture thereof: 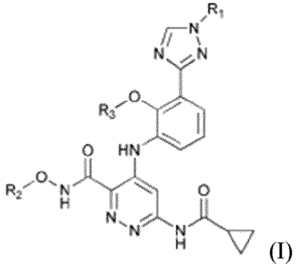 wherein each substituent is as defined in the present invention.

In one embodiment, the present invention provides a pharmaceutical composition comprising the compound, or the pharmaceutically acceptable salt, enantiomer, diastereomer, racemate, solvate, hydrate, polymorph, prodrug or isotopic variant thereof, and the mixture thereof as defined herein, and a pharmaceutically acceptable excipient; wherein preferably, the pharmaceutical composition further comprises other therapeutic agents.

In one embodiment, the present invention provides a use of the compound or the pharmaceutically acceptable salt, enantiomer, diastereomer, racemate, solvate, hydrate, polymorph, prodrug or isotopic variant thereof, and the mixture thereof, or the pharmaceutical composition comprising the same as defined herein in the preparation of a medicament for treating and/or preventing a TYK2-mediated disease.

In one embodiment, the present invention provides a use of the compound or the pharmaceutically acceptable salt, enantiomer, diastereomer, racemate, solvate, hydrate, polymorph, prodrug or isotopic variant thereof, and the mixture thereof, or the pharmaceutical composition comprising the compound as defined herein in the treatment and/or prevention of a TYK2 kinase-mediated disease.

In one embodiment, the present invention provides a method for treating and/or preventing a TYK2 kinase-mediated disease in a subject by using the compound as defined herein, the method comprising administering to the subject the compound or the pharmaceutically acceptable salt, enantiomer, diastereomer, racemate, solvate, hydrate, polymorph, prodrug or isotopic variant thereof, and the mixture thereof, or the pharmaceutical composition comprising the same.

The TYK2 kinase-mediated disease recited in the present invention is selected from an autoimmune disease, a skin disease, an allergic disease, organ rejection, cancer, dry eye disease, myelofibrosis and polycythemia vera. Further, the autoimmune disease is lupus, multiple sclerosis, rheumatoid arthritis, juvenile arthritis, psoriasis, ulcerative colitis, Crohn's disease or autoimmune thyroid disease; the skin disease is psoriasis, a rash or atopic dermatitis; the allergic disease is asthma or rhinitis; the organ transplant rejection is allograft rejection or graft-versus-host disease; and the cancer is renal cancer, liver cancer, pancreatic cancer, gastric cancer, breast cancer, prostate cancer, head and neck cancer, thyroid cancer, lung cancer, glioblastoma, melanoma, lymphoma or leukemia.

In some embodiments, the TYK2 kinase-mediated disease relating to the method is selected from rheumatoid arthritis, psoriasis, ulcerative colitis and Crohn's disease.

### DETAILED DESCRIPTION

### Definitions

The compound, the preparation method therefor and the use thereof of the present invention will be described in further detail below with reference to specific examples. The present invention may be implemented in many different forms and is not limited to the embodiments described herein. On the contrary, these embodiments are provided for the purpose of providing a thorough and complete understanding of the disclosure of the present invention.

Unless otherwise defined, all technical and scientific terms used herein have the same meanings as those commonly understood by a person skilled in the technical field of the present invention. The terms used herein in the description are for the purpose of describing specific examples only and are not intended to limit the present invention. As used herein, the term "and/or" means any one or more of the related listed items and a combination of all items.

The term "alkyl" refers to a saturated hydrocarbon containing a primary (normal) carbon atom, a secondary carbon atom, a tertiary carbon atom, a quaternary carbon atom or a combination thereof. The alkyl is preferably, for example, a C₁-C₆ alkyl, a C₁-C₅ alkyl, a C₁-C₄ alkyl and a C₁-C₃ alkyl. Using "C₁-C₃ alkyl" as an example, the term refers to an alkyl containing 1 to 3 carbon atoms, and each occurrence thereof can be independently a C₁ alkyl, a C₂ alkyl or a C₃ alkyl. Suitable examples include, but are not limited to: methyl (Me, -CH₃), ethyl (Et, -CH₂CH₃), 1-propyl (n-Pr, n-propyl, -CH₂CH₂CH₃) and 2-propyl (i-Pr, i-propyl, -CH(CH₃)₂).

"Alkylene" refers to a divalent group formed by removing another hydrogen from an alkyl, and may be substituted or unsubstituted. In some embodiments, C₁₋₆ alkylenes, C₁₋₄ alkylenes, C₂₋₄ alkylenes and C₁₋₃ alkylenes are preferred. The unsubstituted alkylenes include, but are not limited to: methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Exemplary substituted alkylenes, such as those substituted with one or more alkyl(methyl) groups, include, but are not limited to: substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-, -CH₂CH₂C(CH₃)₂-), etc.

"Alkenyl" is an alkyl as defined herein containing at least one carbon-carbon double bond. In one example, the alkenyl contains 2 to 20 carbon atoms, preferably 2 to 12 carbon atoms, more preferably 2 to 8 carbon atoms, still more preferably 2 to 6 carbon atoms. Non-limiting examples of alkenyls include substituted or unsubstituted vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl or 4-decenyl, etc. When the alkenyl is substituted, there are preferably 1 to 5 substituents, and the substituents are independently selected from F, Cl, Br, I, =O, an alkyl, an alkenyl, an alkynyl, an alkoxy, a hydroxy, a nitro, a cyano and an amino.

"Alkynyl" is an alkyl as defined herein containing at least one carbon-carbon triple bond. In one example, the alkynyl contains 2 to 20 carbon atoms, preferably 2 to 12 carbon atoms, more preferably 2 to 8 carbon atoms, still more preferably 2 to 6 carbon atoms. Non-limiting examples of alkynyls include substituted or unsubstituted ethynyl, 1-propynyl, 2-propynyl, 1-butynyl, 2-butynyl, 3-butynyl, 4-pentynyl, 3-pentynyl, 2-hexynyl, 3-hexynyl, 3-butynyl, 2-heptynyl, 3-heptynyl, 4-heptynyl, 3-octynyl, 3-heptynyl or 4-decynyl, etc. When the alkynyl is substituted, there are preferably 1 to 5 substituents, and the substituents are independently selected from F, Cl, Br, I, =O, an alkyl, an alkenyl, an alkynyl, an alkoxy, a hydroxy, a nitro, a cyano and an amino.

"Carbocyclyl" or "cycloalkyl" refers to a saturated or partially unsaturated cyclic carbon-containing group, such as a 5- to 6-membered saturated carbocyclic ring and a 5- to 6-membered partially unsaturated carbocyclic ring. In one embodiment, the carbocyclyl is a 3- to 4-membered monocyclic, a 3- to 5-membered monocyclic, a 3- to 6-membered monocyclic, a 3- to 7-membered monocyclic, a 3- to 8-membered monocyclic, a 3- to 10-membered monocyclic, a 5- to 8-membered monocyclic, a 5- to 6-membered monocyclic, a 4- to 12-membered bicyclic or a 10- to 15-membered tricyclic ring system. The carbocycle includes bridged or spiro rings. Non-limiting examples of carbocyclyls include cyclopropyl, cyclobutyl, cyclopentyl, cyclohexyl, cyclohexenyl, cycloheptyl, cyclopentenyl, cyclohexadienyl, cycloheptatrienyl, benzocyclopentyl, bicyclo[3.2.1]octyl, bicyclo[5.2.0]nonyl, tricyclo[5.3.1.1]dodecyl, adamantyl or spiro[3.3]heptyl, etc. The carbocyclyls may be optionally substituted. When the carbocyclyl is substituted, there are preferably 1 to 5 substituents, and the substituents are independently selected from F, Cl, Br, I, =O, an alkyl, an alkenyl, an alkynyl, an alkoxy, a hydroxy, a nitro, a cyano and an amino.

The term "halogen" means -F, -Cl, -Br or -I. Further, the term "haloalkyl" refers to an alkyl substituted with a halogen group, wherein the alkyl is as defined above, preferably a C₁₋₆ haloalkyl, a C₁₋₅ haloalkyl, a C₁₋₄ haloalkyl, a C₁₋₃ haloalkyl and a C₁₋₂ haloalkyl.

The term "aryl" refers to an aromatic hydrocarbon group derived from an aromatic ring compound by removing a hydrogen atom, and may be a monocyclic aryl, a condensed-ring aryl, or a polycyclic aryl, and is preferably a 6- to 10-membered aryl. For polycyclic ring species, at least one is an aromatic ring system. Phrases containing this term, such as "5- to 6-membered aryl", means that the aromatic ring system includes 5 to 6 ring atoms. Preferably, the aryl is phenyl.

The term "heteroaryl" refers to an aryl containing a heteroatom, which may be a monocyclic or condensed ring, wherein the heteroatom is independently selected from N, O and S, and the heteroaryl is preferably a 5- to 12-membered heteroaryl, a 5- to 10-membered heteroaryl, preferably a 5- to 8-membered heteroaryl, more preferably a 5- to 6-membered heteroaryl, still more preferably a 5-membered heteroaryl. The heteroaryl includes, but is not limited to, pyrrolyl, furanyl, thienyl, imidazolyl, oxazolyl, isoxazolyl, pyrazolyl, pyridinyl, pyrimidinyl, pyrazinyl, pyridazinyl, quinolyl, isoquinolyl, triazolyl, tetrahydropyrrolyl and thiadiazolyl. In one embodiment, provided is a 5- to 6-membered monocyclic heteroaryl, typically containing one or more, preferably one to three, heteroatoms independently selected from N, O and S. Unless otherwise specified, the "5-membered heteroaryl" is as follows: exemplary 5-membered heteroaryl groups containing one heteroatom include, but are not limited to, pyrrolyl, furanyl and thienyl; exemplary 5-membered heteroaryl groups containing two heteroatoms include, but are not limited to, imidazolyl, pyrazolyl, oxazolinyl, isoxazolinyl, thiazolyl and isothiazolyl; exemplary 5-membered heteroaryl groups containing three heteroatoms include, but are not limited to, thiazolyl, oxadiazolyl and thiadiazolyl; exemplary 5-membered heteroaryl groups containing four heteroatoms include, but are not limited to, tetrazolyl.

"Heterocyclyl" or "heterocycle" refers to a substituted or unsubstituted saturated or partially unsaturated cyclic group containing a heteroatom selected from N, O and S. Furthermore, the term "heterocyclyl" refers to a group having a stable 3- to 10-membered saturated heterocyclic ring system in which one or more atoms constituting a non-aromatic ring are heteroatoms and the rest are carbon. The heteroatoms include, but are not limited to, nitrogen atoms, oxygen atoms, sulfur atoms, etc. The heterocyclyl may be a 3- to 7-membered monocyclic, a 5- to 8-membered monocyclic, a 5- to 6-membered monocyclic, a 4- to 12-membered bicyclic or a 10- to 15-membered tricyclic ring system, preferably a 3- to 10-membered heterocyclyl, and includes at least one, preferably one to four heteroatoms selected from N, O or S. Unless otherwise indicated in the present specification, a heterocycloalkyl group may be a monocyclic system ("monocyclic heterocycloalkyl"), or may be a bicyclic system, a tricyclic system or a polycyclic system with more rings, which may include a fused (condensed), bridged (bridged-ring) or spiro ring system (e.g., a bicyclic system ("bicyclic heterocycloalkyl"). The ring system of the bicyclic heterocycloalkyl may include one or more heteroatoms in one or both rings, and is saturated. Exemplary 3-membered heterocyclyl groups include, but are not limited to, aziridinyl, oxiranyl and thiiranyl, or stereoisomers thereof. Exemplary 4-membered heterocyclyl groups include, but are not limited to, azetidinyl, oxetanyl, thietanyl or isomers and stereoisomers thereof. Exemplary 5-membered heterocyclyl groups include, but are not limited to, tetrahydrofuranyl, tetrahydrothiophenyl, pyrrolidinyl, thiazolidinyl, isothiazolidinyl, oxazolidinyl, isoxazolidinyl, imidazolidinyl, pyrazolidinyl, dioxolanyl, oxathiolanyl, dithiolanyl or isomers and stereoisomers thereof. Exemplary 6-membered heterocyclyl groups include, but are not limited to, piperidinyl, tetrahydropyranyl, thioxanyl, morpholinyl, thiomorpholinyl, dithianyl, dioxanyl, piperazinyl, triazinyl or isomers and stereoisomers thereof. Exemplary 7-membered heterocyclyl groups include, but are not limited to, azepanyl, oxepanyl, thiepanyl and diazepanyl, or isomers and stereoisomers thereof. In one embodiment, a typical heterocyclyl is a 5- to 6-membered monocyclic heterocyclyl containing one or more, preferably one to four, more preferably one to three heteroatoms independently selected from N, O and S. In one embodiment, "heterocycloalkyl" is a 4- to 6-membered heterocycloalkyl, wherein heteroatoms are selected from one or more of N, O and S, and one, two or three heteroatoms are provided.

In various sections of the present invention, linking substituents are described. When the structure clearly requires a linking group, Markush variables listed for that group should be understood as linking groups. For example, if the structure requires a linking group and a Markush group definition for that variable lists "alkyl" or "aryl", it is understood that the "alkyl" or "aryl" represents a linking alkylene group or arylene group, respectively. In some specific structures, when an alkyl group is clearly indicated as a linking group, the alkyl group represents a linked alkylene group. For example, the alkyl in the group "-C₁-C₃ haloalkyl" should be understood as an alkylene.

The term "pharmaceutically acceptable salt" means that the compound can be converted into a corresponding salt by conventional methods, the salt being chemically or physically compatible with other ingredients constituting a pharmaceutical dosage form and physiologically compatible with a receptor. The salt may be an acidic and/or basic salt formed from the compound and an inorganic and/or organic acid and/or an inorganic and/or organic base, and also includes a zwitterionic salt (inner salt), and further includes a quaternary ammonium salt, such as an alkylammonium salt. These salts can be directly obtained during final isolation and purification of the compound. The salts can also be obtained by appropriately mixing the compound of the present invention or a stereoisomer or solvate thereof with a certain amount of an acid or a base. These salts may be precipitated from a solution and collected by filtration, or recovered after evaporation of a solvent, or may be obtained by reaction in an aqueous medium followed by cooling and drying. In particular, the salts are preferably water-soluble, pharmaceutically acceptable, non-toxic acid addition salts, examples of which are salts formed from an amino and an inorganic acid (such as hydrochloric acid, hydrobromic acid, phosphoric acid, sulfuric acid and perchloric acid) or with an organic acid (such as acetic acid, oxalic acid, maleic acid, tartaric acid, citric acid, succinic acid or malonic acid), or salts formed using other conventional methods in the art (such as ion exchange methods). Other pharmaceutically acceptable salts include adipate, alginate, ascorbate, aspartate, benzene sulfonate, benzoate, bisulfate, borate, butyrate, camphorate, camphorsulfonate, citrate, cyclopentyl propionate, diglucosate, dodecyl sulfate, ethanesulfonate, formate, fumarate, gluconate, glycerophosphate, gluconate, hemisulfate, heptanate, hexanoate, hydroiodate, 2-hydroxy-ethanesulfonate, lactoate, lactate, laurate, lauryl sulfate, malate, maleate, malonate, methanesulfonate, 2-naphthalenesulfonate, niacin, nitrate, oleate, oxalate, palmitate, dihydroxynaphthalenate, pectinate, persulfate, 3-phenylpropionate, phosphate, picrate, pivalate, propionate, stearate, succinate, sulfate, tartrate, thiocyanate, *p*-toluenesulfonate, undecanoate, valerate, etc. Additional pharmaceutically acceptable salts may further include, where appropriate, salts derived from suitable bases, including alkali metal salts, alkaline earth metal salts and ammonium salts. Representative alkali metal or alkaline earth metal salts include a sodium salt, a lithium salt, a potassium salt, a calcium salt, a magnesium salt, etc. Additional pharmaceutically acceptable salts include, where appropriate, salts formed using counterions such as halides, hydroxide, carboxylate, sulfate, phosphate, nitrate, lower alkyl sulfonates and aryl sulfonates with non-toxic ammonium, quaternary ammonium and amine cations.

The term "solvate" may also be called "solvate compound" or "solvate", and refers to a compound containing solvent molecules, where the solvent molecules can bind to the compound molecules in ways including coordination bonds, covalent bonds, van der Waals forces, ionic bonds, hydrogen bonds, etc. Common solvents include water, methanol, ethanol, acetic acid, DMSO, THF, diethyl ether, etc. The compounds described herein can be prepared, e.g., in crystalline forms, and can be solvated. Suitable solvates include pharmaceutically acceptable solvates and further include stoichiometric and non-stoichiometric solvates. In some cases, the solvate will be capable of being isolated, for example, when one or more solvent molecules are incorporated into crystal lattices of a crystalline solid. The "solvate" includes solvates in solution and isolatable solvates. Representative solvates include hydrates, ethanolates and methanolates.

The term "hydrate" refers to a compound binding to water. Usually, the ratio of the number of water molecules contained in a hydrate of a compound to the number of molecules of the compound in the hydrate is determinate. Thus, a hydrate of a compound can be represented by, for example, a general formula R·xH₂O, where R is the compound, and x is a number greater than zero. A given compound may form more than one type of hydrate, including, for example, a monohydrate (x is 1), lower hydrates (x is a number greater than 0 and less than 1, for example, a hemihydrate (R·0.5H₂O)) and polyhydrates (x is a number greater than 1, for example, a dihydrate (R·2H₂O) and a hexahydrate (R·6H₂O)).

The term "prodrug" refers to any compound that, when administered to an organism, produces a drug, i.e., an active ingredient, as a result of a spontaneous chemical reaction, an enzyme-catalyzed chemical reaction, photolysis and/or a metabolic chemical reaction. Prodrugs are thus covalently modified analogs or latent forms of therapeutically active compounds. Suitable examples include, but are not limited to: carboxylates, carbonates, phosphates, nitrates, sulfates, sulfones, sulfoxides, amides, carbamates, azo compounds, phosphoramides, glucosides, ethers, acetals and other forms of the compound.

The present invention further includes isotopically labeled compounds (isotopic variants), which are equivalent to those general formula or specific compounds described herein except that one or more atoms are replaced by atoms having an atomic mass or mass number different from the atomic mass or mass number usually found in nature. Examples of isotopes that can be introduced into the compounds of the present invention include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively, preferably ²H (i.e., deuterium, D). The compounds of the present invention which contain the aforementioned isotopes and/or other isotopes of other atoms, prodrugs of the compounds, and pharmaceutically acceptable salts of the compounds or the prodrugs are all within the scope of the present invention. Certain isotopically labeled compounds of the present invention, such as those into which radioactive isotopes such as ³H and ¹⁴C are incorporated, are useful in drug and/or substrate tissue distribution assays. Tritium (i.e., ³H) and carbon-14 (i.e., ¹⁴C) isotopes are particularly preferred for the ease of preparation and detectability. Additionally, substitution with heavier isotopes such as deuterium (i.e., ²H) may be preferred in some cases since substituted compounds may provide therapeutic advantages by virtue of greater metabolic stability, such as prolonged in-vivo half-life or reduced dosage requirements. Isotopically labeled compounds of the present invention and prodrugs thereof can generally be prepared by substituting readily available isotopically labeled reagents for non-isotopically labeled reagents when carrying out the processes disclosed in the following procedures and/or examples and preparative examples.

The compound of the present invention includes one or more asymmetric centers, and thus may have a plurality of stereoisomer forms, e.g., enantiomer and/or diastereomer forms. For example, the compound of the present invention may be a separate enantiomer, diastereomer or geometric isomer (e.g., *cis-* and trans-isomers), or may be in the form of a mixture of stereoisomers, including a mixture of racemates and a mixture rich in one or more stereoisomers. Isomers can be separated from the mixture by methods known to those skilled in the art, including: chiral high pressure liquid chromatography (HPLC) and formation and crystallization of chiral salts. Alternatively, preferred isomers may be prepared by asymmetric synthesis.

"Optional" or "optionally" means that a subsequently described event or circumstance may, but not necessarily, occur, including cases where the event or circumstance does or does not occur. For example, "an aryl is optionally substituted with an alkyl" means that the alkyl may, but not necessarily, be present, and the term includes instances where the aryl is substituted with an alkyl and instances where the aryl is not substituted with an alkyl.

"Pharmaceutically acceptable excipient" refers to a pharmaceutically acceptable material, composition or agent, such as a liquid or solid filler, a diluent, an excipient, a solvent or an encapsulating material. As used herein, the term "pharmaceutically acceptable excipient" includes buffers, sterile water for injection, solvents, dispersion media, coatings, antibacterial and antifungal agents, isotonicity agents, absorption retarders, etc., compatible with pharmaceutical administration. Each excipient needs to be "pharmaceutically acceptable" in the sense of being compatible with other ingredients in a formulation and not harmful to the patient. Suitable examples include, but are not limited to: (1) sugars, such as lactose, glucose and sucrose; (2) starches, such as corn starch, potato starch and substituted or unsubstituted β-cyclodextrin; (3) cellulose and derivatives thereof, such as sodium carboxymethyl cellulose, ethyl cellulose and cellulose acetate; (4) powdered tragacanth gum; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) phosphate buffers; and (21) other non-toxic compatible substances used in drug formulations.

The term "polymorph" refers to a crystalline form of a compound (or a salt, hydrate or solvate thereof) of a particular crystal packing arrangement. All polymorphs have the same elemental composition. Different crystalline forms typically have different X-ray diffraction patterns, infrared spectra, melting points, densities, hardness, crystal shapes, optoelectronic properties, stability and solubility. Recrystallization solvent, rate of crystallization, storage temperature and other factors can cause one crystalline form to dominate. Various polymorphs of a compound can be prepared by crystallization under different conditions.

Unless defined otherwise, all technical and scientific terms used herein have the standard meanings in the art to which the claimed subject matter belongs. If there are multiple definitions for a term, the definition in this document shall prevail. It should be understood that the singular forms used in the present invention, such as "a" and "an", include plural references, unless otherwise specified.

In addition, the terms "comprise" and "include" are open-ended limitations rather than closed-ended ones, that is, they include the content specified in the present invention but do not exclude other aspects.

Unless otherwise specified, the present invention uses conventional methods such as mass spectrometry and nuclear magnetic resonance to identify compounds, and for steps and conditions, reference can be made to conventional operating steps and conditions in the art.

Unless otherwise indicated, the present invention uses standard nomenclature and standard laboratory procedures and techniques of analytical chemistry, synthetic organic chemistry and optics. In some cases, standard techniques are used for chemical synthesis, chemical analysis and light-emitting device performance testing.

In addition, it should be noted that, unless otherwise clearly stated, the description "... respectively independently" used in the present invention should be understood in a broad sense, meaning that each entity described is independent of each other and can independently be the same specific group or different specific groups. In more detail, the description "... respectively independently" may mean that specific options expressed by the same symbol do not affect each other among different groups, or may mean that specific options expressed by the same symbols do not affect each other in the same group.

Specifically, the present invention relates to the following technical solutions.

In one embodiment, the present invention relates to a compound represented by general formula (I), or a pharmaceutically acceptable salt, enantiomer, diastereomer, racemate, solvate, hydrate, polymorph, prodrug or isotopic variant thereof, and a mixture thereof: 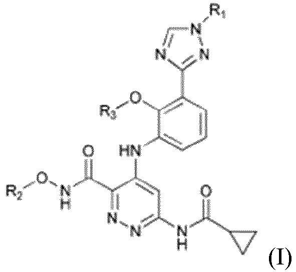 wherein
R₁ is selected from H, a C₁₋₆ haloalkyl, a C₁₋₆ alkylene-ORₐ, a C₁₋₆ alkylene-SRₐ, a C₁₋₆ alkylene-NR_{b}R_{c}, a C₁₋₆ alkylene-C(O)Rₐ, a C₁₋₆ alkylene-C(O)ORₐ, a C₁₋₆ alkylene-C(O)NR_{b}R_{c}, a C₁₋₆ alkylene-OC(O)Rₐ, a C₁₋₆ alkylene-NR_{b}C(O)Rₐ, a C₁₋₆ alkylene-S(O)ₘRₐ, a C₁₋₆ alkyleneS(O)ₘNR_{b}R_{c} or a C₁₋₆ alkylene-NR_{b}S(O)ₘRₐ, and the group is optionally substituted with one or more deuteriums until fully deuterated;
R₂ is selected from a C₁₋₆ alkyl, a C₁₋₆ haloalkyl, a C₂₋₆ alkenyl, a C₂₋₆ alkynyl, a C₃₋₇ cycloalkyl, a 3- to 7-membered heterocyclyl, a C₆₋₁₀ aryl or a 5- to 10-membered heteroaryl, and the group is optionally substituted with one or more deuteriums until fully deuterated;
R₃ is selected from H, a C₁₋₆ alkyl or a C₁₋₆ haloalkyl, and the group is optionally substituted with one or more deuteriums until fully deuterated;
each group of R₁, R₂ and R₃ is optionally substituted with 1, 2, 3 or 4 R;
wherein Rₐ, R_{b} and R_{c} are independently selected from H, a C₁₋₆ alkyl, a C₁₋₆ haloalkyl, a C₂₋₆ alkenyl, a C₂₋₆ alkynyl, a C₃₋₇ cycloalkyl, a 3- to 7-membered heterocyclyl, a C₆₋₁₀ aryl or a 5-to 10-membered heteroaryl;
m is 1 or 2; and
R is selected from H, D, a halogen, CN, a C₁₋₆ alkyl or a C₁₋₆ haloalkyl.

In a more specific embodiment, the present invention provides the compound represented by formula (I), or the pharmaceutically acceptable salt, enantiomer, diastereomer, racemate, solvate, hydrate, polymorph, prodrug or isotopic variant thereof, and the mixture thereof, wherein R₁ is selected from H, a C₁₋₆ haloalkyl, a C₁₋₆ alkylene-ORₐ, a C₁₋₆ alkylene-SRₐ, a C₁₋₆ alkylene-NR_{b}R_{c}, a C₁₋₆ alkylene-C(O)Rₐ, a C₁₋₆ alkylene-C(O)ORₐ, a C₁₋₆ alkylene-C(O)NR_{b}R_{c}, a C₁₋₆ alkylene-OC(O)Rₐ or a C₁₋₆ alkylene-NR_{b}C(O)Rₐ, and the group is optionally substituted with one or more deuteriums until fully deuterated; preferably, R₁ is selected from H, a C₁₋₆ haloalkyl, a C₁₋₆ alkylene-ORₐ, a C₁₋₆ alkylene-SRₐ, a C₁₋₆ alkylene-NR_{b}Rₑ, a C₁₋₆ alkylene-C(O)ORₐ or a C₁₋₆ alkylene-C(O)NR_{b}R_{c}, and the group is optionally substituted with one or more deuteriums until fully deuterated; preferably, R₁ is selected from H, a C₁₋₆ haloalkyl, a C₁₋₆ alkylene-OH, a C₁₋₆ alkylene-SH, a C₁₋₆ alkylene-NH₂, a C₁₋₆ alkylene-C(O)OH or a C₁₋₆ alkylene-C(O)NH₂, and the group is optionally substituted with one or more deuteriums until fully deuterated; preferably, R₁ is selected from H or a C₁₋₆ haloalkyl, and the group is optionally substituted with one or more deuteriums until fully deuterated; and preferably, R₁ is a C₁₋₆ alkylene-ORₐ, wherein Rₐ is selected from a C₁₋₆ alkyl or a C₁₋₆ haloalkyl.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein R₂ is selected from a C₁₋₆ alkyl, a C₁₋₆ haloalkyl, a C₂₋₆ alkenyl, a C₂₋₆ alkynyl, a C₃₋₇ cycloalkyl or a 3- to 7-membered heterocyclyl, and the group is optionally substituted with one or more deuteriums until fully deuterated; preferably, R₂ is selected from a C₁₋₆ alkyl, a C₁₋₆ haloalkyl, a C₃₋₇ cycloalkyl or a 3- to 7-membered heterocyclyl, and the group is optionally substituted with one or more deuteriums until fully deuterated; and preferably, R₂ is selected from a C₁₋₆ alkyl or a C₃₋₇ cycloalkyl, and the group is optionally substituted with one or more deuteriums until fully deuterated.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein R₃ is selected from a C₁₋₆ alkyl or a C₁₋₆ haloalkyl, and the group is optionally substituted with one or more deuteriums until fully deuterated; and preferably, R₃ is methyl, which is optionally substituted with one or more deuteriums until fully deuterated.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein
R₁ is selected from H, a C₁₋₆ haloalkyl, a C₁₋₆ alkylene-ORₐ, a C₁₋₆ alkylene-SRₐ or a C₁₋₆ alkylene-C(O)ORₐ, and the group is optionally substituted with one or more deuteriums until fully deuterated;
R₂ is selected from a C₁₋₆ alkyl, a C₁₋₆ haloalkyl, a C₃₋₇ cycloalkyl or a 3- to 7-membered heterocyclyl, and the group is optionally substituted with one or more deuteriums until fully deuterated;
R₃ is selected from H, a C₁₋₆ alkyl or a C₁₋₆ haloalkyl, and the group is optionally substituted with one or more deuteriums until fully deuterated;
each group of R₁, R₂ and R₃ is optionally substituted with 1, 2, 3 or 4 R;
wherein Rₐ is selected from H, a C₁₋₆ alkyl or a C₁₋₆ haloalkyl; and
R is selected from H, D, a halogen, CN, a C₁₋₆ alkyl or a C₁₋₆ haloalkyl.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein
R₁ is selected from H, a C₁₋₄ haloalkyl, a C₁₋₄ alkylene-ORₐ or a C₁₋₄ alkylene-C(O)OH, and the group is optionally substituted with one or more deuteriums until fully deuterated;
R₂ is selected from a C₁₋₄ alkyl, a C₁₋₄ haloalkyl or a C₃₋₇ cycloalkyl, and the group is optionally substituted with one or more deuteriums until fully deuterated; and
R₃ is selected from a C₁₋₄ alkyl or a C₁₋₄ haloalkyl, and the group is optionally substituted with one or more deuteriums until fully deuterated;
wherein Rₐ is selected from H, a C₁₋₄ alkyl or a C₁₋₄ haloalkyl.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein
R₁ is a C₁₋₄ alkylene-ORₐ, which is optionally substituted with one or more deuteriums until fully deuterated;
R₂ is selected from a C₁₋₄ alkyl, a C₁₋₄ haloalkyl or a C₃₋₇ cycloalkyl, and the group is optionally substituted with one or more deuteriums until fully deuterated; preferably a C₁₋₄ alkyl, which is optionally substituted with one or more deuteriums until fully deuterated; and
R₃ is selected from a C₁₋₄ alkyl or a C₁₋₄ haloalkyl, and the group is optionally substituted with one or more deuteriums until fully deuterated;
wherein Rₐ is selected from a C₁₋₄ alkyl or a C₁₋₄ haloalkyl.

In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein
R₁ is selected from H, a C₁₋₄ haloalkyl, a C₁₋₄ alkylene-OH or a C₁₋₄ alkylene-C(O)OH, and the group is optionally substituted with one or more deuteriums until fully deuterated;
R₂ is selected from a C₁₋₄ alkyl, a C₁₋₄ haloalkyl or a C₃₋₇ cycloalkyl, and the group is optionally substituted with one or more deuteriums until fully deuterated; preferably a C₁₋₄ alkyl, which is optionally substituted with one or more deuteriums until fully deuterated; and
R₃ is selected from a C₁₋₄ alkyl or a C₁₋₄ haloalkyl, and the group is optionally substituted with one or more deuteriums until fully deuterated.

In a more specific embodiment, the present invention provides the compound represented by formula (I) above or the pharmaceutically acceptable salt, enantiomer, diastereomer, racemate, solvate, hydrate, polymorph, prodrug or isotopic variant thereof, and the mixture thereof, wherein
R₁ is selected from H, a C₁₋₆ alkylene-ORₐ and a C₁₋₆ alkylene-SRₐ, and the group is optionally substituted with one or more deuteriums until fully deuterated;
R₂ is selected from a C₁₋₆ alkyl and a C₁₋₆ haloalkyl, and the group is optionally substituted with one or more deuteriums until fully deuterated;
R₃ is selected from H, a C₁₋₆ alkyl or a C₁₋₆ haloalkyl, and the group is optionally substituted with one or more deuteriums until fully deuterated;
each group of R₁, R₂ and R₃ is optionally substituted with 1, 2, 3 or 4 R;
wherein Rₐ is selected from H, a C₁₋₆ alkyl and a C₁₋₆ haloalkyl; and
R is selected from H, D, a halogen, CN, a C₁₋₆ alkyl or a C₁₋₆ haloalkyl.

In a more specific embodiment, the present invention provides the compound represented by formula (I) above or the pharmaceutically acceptable salt, enantiomer, diastereomer, racemate, solvate, hydrate, polymorph, prodrug or isotopic variant thereof, and the mixture thereof, wherein
R₁ is selected from H and a C₁₋₆ alkylene-ORₐ, and the group is optionally substituted with one or more deuterium groups until fully deuterated;
R₂ is selected from a C₁₋₆ alkyl and a C₁₋₆ haloalkyl; and
R₃ is a C₁₋₆ alkyl, and the group is optionally substituted with one or more deuteriums until fully deuterated;
wherein Rₐ is selected from H and a C₁₋₆ alkyl.

In a more specific embodiment, the present invention provides the compound represented by formula (I) above or the pharmaceutically acceptable salt, enantiomer, diastereomer, racemate, solvate, hydrate, polymorph, prodrug or isotopic variant thereof, and the mixture thereof, wherein
R₁ is selected from H and a C₁₋₄ alkylene-ORₐ, and the group is optionally substituted with one or more deuterium groups until fully deuterated;
R₂ is a C₁₋₄ alkyl, and the group is optionally substituted with one or more deuteriums until fully deuterated; and
R₃ is a C₁₋₄ alkyl, and the group is optionally substituted with one or more deuteriums until fully deuterated;
wherein Rₐ is a C₁₋₄ alkyl. In a more specific embodiment, the present invention provides the compound represented by formula (I) above, or the pharmaceutically acceptable salt, enantiomer, diastereomer, racemate, solvate, hydrate, polymorph, prodrug or isotopic variant thereof, and the mixture thereof, wherein the compound is selected from:

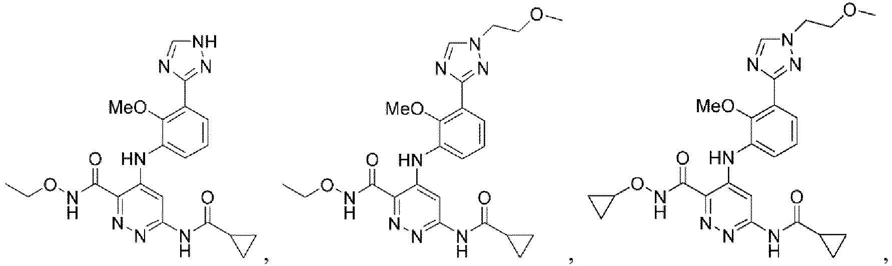

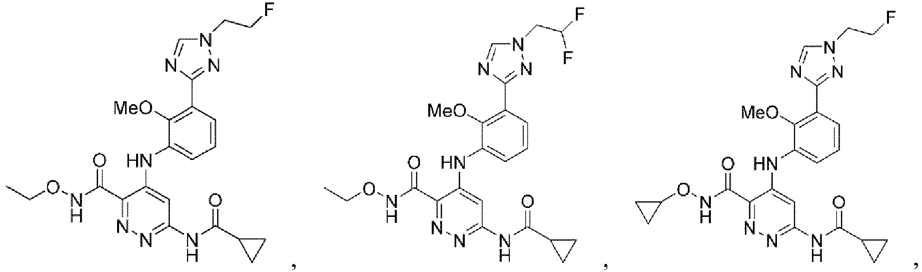

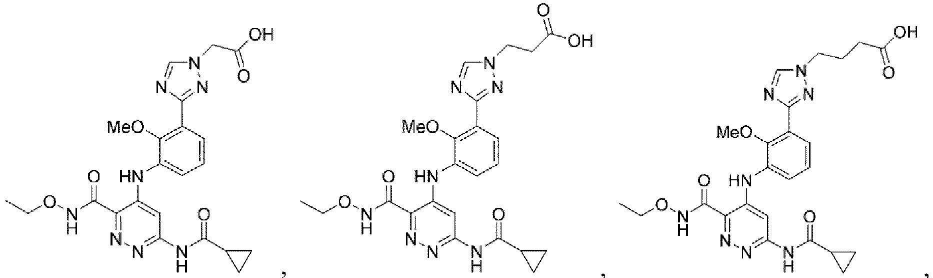

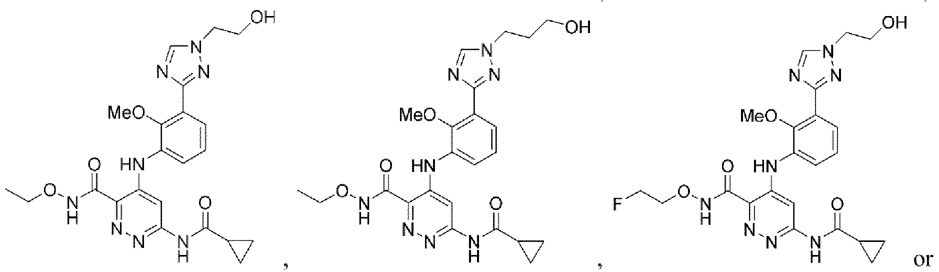

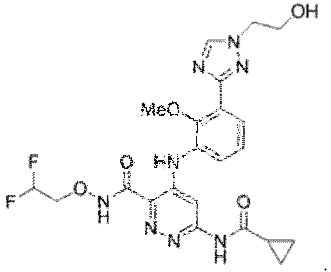

In one embodiment, the present invention provides a pharmaceutical composition including the compound, or the pharmaceutically acceptable salt, enantiomer, diastereomer, racemate, solvate, hydrate, polymorph, prodrug or isotopic variant thereof, and the mixture thereof as defined herein, and a pharmaceutically acceptable excipient. Preferably, the pharmaceutical composition further includes other therapeutic agents.

In one embodiment, the present invention provides a use of the compound or the pharmaceutically acceptable salt, enantiomer, diastereomer, racemate, solvate, hydrate, polymorph, prodrug or isotopic variant thereof as defined herein, and the mixture thereof, or the pharmaceutical composition including the same in the preparation of a medicament for treating and/or preventing a TYK2 kinase-mediated disease.

In one embodiment, the present invention provides a use of the compound or the pharmaceutically acceptable salt, enantiomer, diastereomer, racemate, solvate, hydrate, polymorph, prodrug or isotopic variant thereof, and the mixture thereof, or the pharmaceutical composition including the compound as defined herein in the treatment and/or prevention of a TYK2 kinase-mediated disease.

In one embodiment, the present invention provides a method for treating and/or preventing a TYK2 kinase-mediated disease in a subject by using the compound as defined herein, the method including administering to the subject the compound or the pharmaceutically acceptable salt, enantiomer, diastereomer, racemate, solvate, hydrate, polymorph, prodrug or isotopic variant thereof, and the mixture thereof, or the pharmaceutical composition including the same.

The TYK2 kinase-mediated disease recited in the present invention is selected from an autoimmune disease, a skin disease, an allergic disease, organ rejection, cancer, dry eye disease, myelofibrosis and polycythemia vera. Further, the autoimmune disease is lupus, multiple sclerosis, rheumatoid arthritis, juvenile arthritis, psoriasis, ulcerative colitis, Crohn's disease or autoimmune thyroid disease; the skin disease is psoriasis, a rash or atopic dermatitis; the allergic disease is asthma or rhinitis; the organ transplant rejection is allograft rejection or graft-versus-host disease; and the cancer is renal cancer, liver cancer, pancreatic cancer, gastric cancer, breast cancer, prostate cancer, head and neck cancer, thyroid cancer, lung cancer, glioblastoma, melanoma, lymphoma or leukemia.

In some embodiments, the TYK2 kinase-mediated disease relating to the method is selected from rheumatoid arthritis, psoriasis, ulcerative colitis and Crohn's disease.

Those skilled in the art will understand that, without departing from common general knowledge in the art, the various preferred conditions described above can be arbitrarily combined to obtain various preferred embodiments of the present invention.

### Administration

The compound represented by (I) of the present invention may be administered by any means appropriate to the state of a disease to be treated. The means may depend on the need for site-specific treatment or the amount of a medicament to be delivered. Although other modes of delivery are contemplated, topical administration is generally preferred for skin-related diseases, and systemic treatment is preferred for cancerous or precancerous disease states. For example, the compound may be delivered by: oral administration, e.g., in the form of tablets, capsules, granules, powders or liquid preparations (including syrups); topical administration, e.g., in the form of solutions, suspensions, gels or ointments; sublingual administration; transbuccal administration; parenteral administration, e.g., by subcutaneous, intravenous, intramuscular or intrasternal injection or infusion techniques (e.g., in the form of sterile injectable aqueous or non-aqueous solutions or suspensions); nasal administration, e.g., by inhalation of sprays; topical administration, e.g., in the form of creams or ointments; rectal administration, e.g., in the form of suppositories; or liposomes. Unit dose formulations containing a non-toxic pharmaceutically acceptable carrier or diluent may be administered. The compounds may be administered in a form suitable for immediate release or extended release. Immediate release or extended release can be achieved using suitable pharmaceutical compositions or, particularly in the case of extended release, using devices such as subcutaneous implants or osmotic pumps.

### Exemplary compositions for topical administration include topical carriers.

Exemplary compositions for oral administration include suspensions, which may contain, for example, microcrystalline cellulose to impart bulk, alginic acid or sodium alginate as a suspending agent, methylcellulose as a tackifier, and a sweetener or a flavoring agent such as those known in the art; and immediate release tablets, which may contain, for example, microcrystalline cellulose, dicalcium phosphate, starch, magnesium stearate and/or lactose and/or other excipients, binders, extenders, disintegrants, diluents and lubricants, such as those known in the art. The compound of the present invention may also be delivered orally by sublingual and/or buccal administration, for example, using molded, compressed or freeze-dried tablets. Exemplary compositions may include fast dissolving diluents, such as mannitol, lactose, sucrose and/or cyclodextrins. These formulations may also include high molecular weight excipients such as cellulose (AVICEL^{®}) or polyethylene glycol (PEG); excipients to aid mucoadhesion, such as hydroxypropylcellulose (HPC), hydroxypropylmethylcellulose (HPMC), sodium carboxymethylcellulose (SCMC) and/or maleic anhydride copolymers (e.g., GANTREZ^{®}); and agents for controlled release, such as polyacrylic acid copolymers (e.g., CARBOPOL 934^{®}). Lubricants, glidants, flavoring agents, coloring agents and stabilizers may also be added to facilitate preparation and use.

Exemplary compositions for nasal aerosol or inhalation administration include solutions, which may contain, for example, benzyl alcohol or other suitable preservatives, absorption promoters for enhancing absorption and/or bioavailability and/or other solubilizing or dispersing agents such as those known in the art.

Exemplary compositions for parenteral administration include injectable solutions or suspensions, which may contain, for example, suitable non-toxic parenterally acceptable diluents or solvents, such as mannitol, 1,3-butanediol, water, Ringer's solution, isotonic sodium chloride solution, or other suitable dispersing or wetting agents and suspending agents, including synthetic mono- or diglycerides and fatty acids, including oleic acid.

Exemplary compositions for rectal administration include suppositories, which may contain, for example, suitable non-irritating excipients such as cocoa butter, synthetic glycerides or polyethylene glycols, these excipients being solid at normal temperature but liquefying and/or dissolving in the rectal cavity to release a medicament.

The therapeutically effective amount of the compound of the present invention can be determined by those skilled in the art, and includes exemplary doses of about 0.05-1000 mg/kg, 1-1000 mg/kg, 1-50 mg/kg, 5-250 mg/kg and 250-1000 mg/kg body weight of active compounds per day for mammals, which can be administered as a single dose or as individual divided doses (e.g., 1 to 4 times per day). It should be understood that the specific dosage level and administration frequency for any particular subject may vary and will depend on a variety of factors, including the activity of a specific compound used, the metabolic stability and duration of action of the compound, the species, age, weight, general health, sex and diet of the subject, the mode and time of administration, the rate of excretion, drug combinations and the severity of a particular disease state. Preferred subjects for treatment include animals, most preferably mammalian species, such as humans and domestic animals like dogs, cats, horses, etc. Thus, when the term "patient" is used herein, the term is meant to include all subjects, most preferably mammalian species suffering from TYK2 kinase-mediated diseases.

### Examples

The materials and reagents used herein are either commercially available or prepared by synthetic methods generally known in the art.

### Example 1

### 6-(Cyclopropylcarboxamido)-N-ethoxy-4-((2-methoxy-3-(1H-1,2,4-triazol-3-yl)phenyl)amino)pyridazine-3-carboxamide (Compound 1)

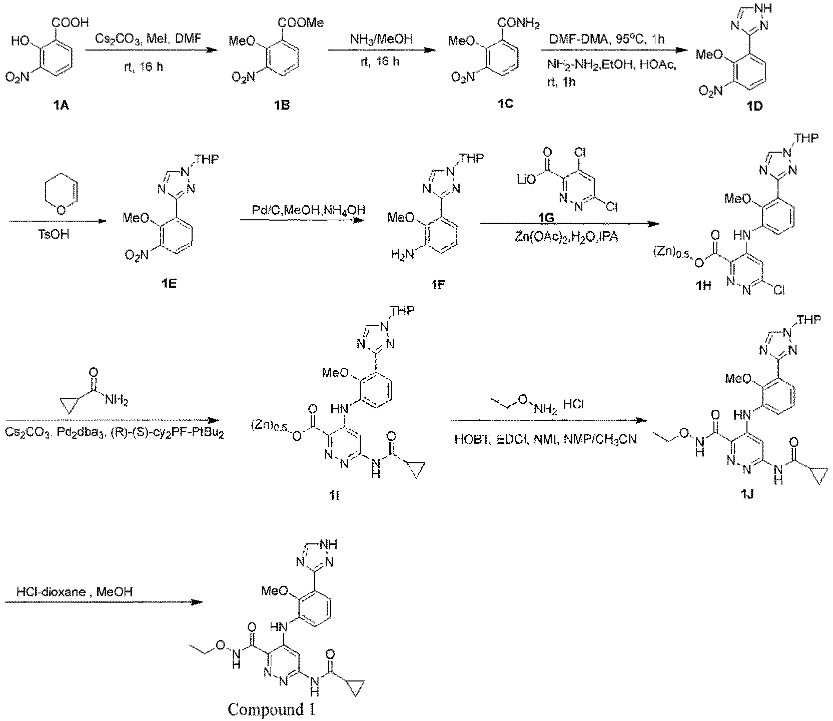

### Step 1: Methyl 2-methoxy-3-nitrobenzoate

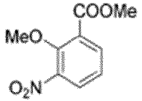

3-Nitrosalicylic acid (15 g, 81.91 mmol) and cesium carbonate (106.756 g, 327.65 mmol) were dissolved in *N,N*-dimethylformamide (300 mL), iodomethane (58.54 g, 409.57 mmol) was added to the reaction system, and the reaction solution was stirred and allowed to react at room temperature for 16 hours. The reaction was stopped, and water (1.5 L) was added to the reaction solution for quenching. After filtration, the filter cake was dried to obtain the title compound (12.886 g, yield: 74.5%, white solid).

MS (ESI): m/z 212.1 [M+H]+.

### Step 2: 2-Methoxy-3-nitrobenzamide

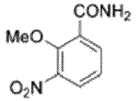

Methyl 2-methoxy-3-nitrobenzoate (6.66 g, 31.54 mmol) was dissolved in methanol (36.63 mL), aqueous ammonia (27.3 mL) was added, and the mixture was allowed to react at room temperature for 16 hours. The reaction was stopped, and the reaction solution was concentrated under reduced pressure and then separated and purified by column chromatography (silica gel, dichloromethane: methanol = 95:5) to obtain the title compound (5.2 g, yield: 84%, yellow solid).

MS (ESI): m/z 197.0 [M+H]+.

### Step 3: 3-(2-Methoxy-3-nitrophenyl)-1H-1,2,4-triazole

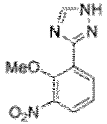

2-Methoxy-3-nitrobenzamide (5.2 g, 26.53 mmol) was dissolved in *N,N-*dimethylformamide dimethyl acetal (67.6 mL), and the mixture was allowed to react at 95°C for 1 h. The reaction solution was spun in vacuo to remove the solvent, and ethanol (13 mL) was used for dissolution to obtain a crude product. Ethanol (110 mL) and acetic acid (26 mL) were stirred at 0°C for 5 minutes, and hydrazine hydrate (13.18 mL) was added thereto. After the mixture was stirred and allowed to react for 15 minutes, the crude product solution was added, and the mixture was warmed to room temperature, and was stirred and allowed to react for one hour. The reaction was stopped, the reaction solution was concentrated under reduced pressure, then extracted using added ethyl acetate (451 mL), and then washed twice using saturated sodium bicarbonate solution (451 mL), washed using saturated brine, dried over anhydrous sodium sulfate and then concentrated under reduced pressure. The crude product was separated and purified by column chromatography (silica gel, dichloromethane:methanol = 9:1) to obtain the title compound (3.71 g, yield: 63.5%, yellow solid).

MS (ESI): m/z 221.1 [M+H]+.

### Step 4: 3-(2-Methoxy-3-nitrophenyl)-1-(tetrahydro-2H-pyran-2-yl)-1H-1,2,4-triazole

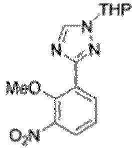

3-(2-Methoxy-3-nitrophenyl)-1*H*-1,2,4-triazole (1.3 g, 5.9 mmol), 3,4-dihydro-2*H*-pyran (2.5 g, 29.52 mmol) and 4-methylbenzenesulfonic acid (224.61 mg, 1.18 mmol) were dissolved in tetrahydrofuran (52 mL), and the reaction solution was stirred at 80°C for 16 hours. The reaction was stopped, water (50 mL) was added to the reaction solution for quenching, and then extraction was performed three times using ethyl acetate (100 mL). The merged organic phase was washed with saturated brine, dried over anhydrous sodium sulfate, concentrated under reduced pressure, and then separated and purified by column chromatography (silica gel, petroleum ether:ethyl acetate = 1:1) to obtain the title compound (1.8 g, yield: 100%, yellow oily matter).

MS (ESI): m/z 305.1 [M+H]+.

### Step 5: 2-Methoxy-3-(1-(tetrahydro-2H-pyran-2-yl)-1H-1,2,4-triazol-3-yl)aniline

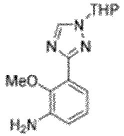

3-(2-Methoxy-3-nitrophenyl)-1-(tetrahydro-2*H*-pyran-2-yl)-1*H*-1,2,4-triazole (1.8 g, 5.92 mmol) was dissolved in methanol (50 mL), aqueous ammonia (0.85 mL) was added, and then palladium/carbon (251.8 mg, 2.4 mmol) was added in batches. After replacement with hydrogen, the mixture was allowed to react at room temperature for 16 hours. The reaction was stopped. The reaction solution was filtered, and the filtrate was concentrated under reduced pressure, and then separated and purified by column chromatography (silica gel, dichloromethane: methanol = 100:1) to obtain the title compound (1.572 g, yield: 97%, yellow oily matter).

MS (ESI): m/z 275.1 [M+H]+.

### Step 6: ((6-Chloro-4-((2-methoxy-3-(1-(tetrahydro-2H-pyran-2-yl)-1H-1,2,4-triazol-3-yl)phenyl)amino)pyridazine-3-carbonyl)oxy)zinc (0.5)

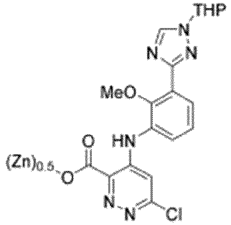

2-Methoxy-3-(1-(tetrahydro-2*H*-pyran-2-yl)-1*H*-1,2,4-triazol-3-yl)aniline (400 mg, 1.46 mmol), lithium 4,6-dichloropyridazine-3-carboxylate (348.21 mg, 1.75 mmol) and zinc acetate (321.08 mg, 1.75 mmol) were dissolved in a 10 mL solvent of water:isopropanol = 7:1. After replacement with nitrogen, the mixture was allowed to react at 65°C for 16 hours. The reaction was stopped, 15 mL of water was added to the reaction solution, and the mixture was stirred for one hour and then filtered by suction and washed using tetrahydrofuran (0.5 mL). The filter cake was dried to obtain the title compound (365 mg, yield: 54%, white solid).

MS (ESI): m/z 431.1 [M+H]+.

### Step 7: ((6-(Cyclopropanecarboxamido)-4-((2-methoxy-3-(1-(tetrahydro-2H-pyran-2-yl)-1H-1,2,4-triazol-3-yl)phenyl)amino))pyridazine-3-carbonyl)oxy)zinc (0.5)

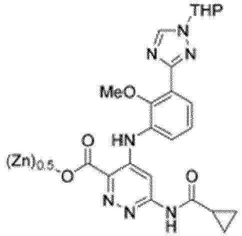

((6-Chloro-4-((2-methoxy-3-(1-(tetrahydro-2*H*-pyran-2-yl)-1*H*-1,2,4-triazol-3-yl)phenyl)amino)pyridazine-3-carbonyl)oxy)zinc (0.5) (335 mg, 0.724 mmol), cyclopropylcarboxamide (308.18 mg, 3.62 mmol), (2*R*)*-*1*-*[(1*R*)-1-[bis(1,1-di-*tert-*butyl)phosphino]ethyl]-2-(dicyclohexylphosphino)ferrocene (120.49 mg, 0.217 mmol), tris[dibenzylideneacetone]dipalladium (132.64 mg, 0.145 mmol) and cesium carbonate (471.97 mg, 1.45 mmol) were dissolved in *N,N*-dimethylacetamide (5 mL). After replacement with nitrogen, the mixture was allowed to react at 140°C for 1 hour. The reaction was stopped, the reaction solution was filtered, and then the filtrate was separated and purified by reverse phase column chromatography (C18, water/0.1% TFA:acetonitrile = 70:30) to obtain the title compound (216 mg, yield: 58%, yellow solid).

MS (ESI): m/z 480.4 [M+H]+.

### Step 8: 6-(Cyclopropylcarboxamido)-N-ethoxy-4-((2-methoxy-3-(1-(tetrahydro-2H-pyran-2-yl)-1H-1,2,4-triazol-3-yl)phenyl)amino)pyridazine-3-carboxamide

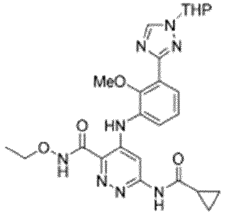

N-Methylpyrrolidone (3 mL) and acetonitrile (3 mL) were added to a reaction flask, and then ((6-(cyclopropanecarboxamido)-4-((2-methoxy-3-(1-(tetrahydro-2*H*-pyran-2-yl)-1*H*-1,2,4-triazol-3-yl)phenyl)amino))pyridazine-3-carbonyl)oxy)zinc (0.5) (100 mg, 0.196 mmol), *O-*ethylhydroxylamine hydrochloride (28.62 mg, 0.293 mmol) and *N*-methylimidazole (48.18 mg, 0.587 mmol) were added. After replacement with nitrogen, the mixture was allowed to react in an oil bath at 65°C for 15 minutes, and finally 1-hydroxybenzotriazole (52.87 mg, 0.39 mmol) and 1-(3-dimethylaminopropyl)-3-ethylcarbodiimide hydrochloride (75 mg, 0.39 mmol) were added. After replacement with nitrogen, the mixture was allowed to react in an oil bath at 65°C for 16 hours. The reaction was stopped, water (10 mL) was added to the reaction solution for quenching, and then extraction was performed using ethyl acetate (10 mL × 3). The merged organic phase was washed with saturated brine, dried over anhydrous sodium sulfate and filtered, and then the filtrate was spun in vacuo to remove the solvent to obtain the title compound (124 mg, brown oily matter).

MS (ESI): m/z 523.2 [M+H]+.

### Step 9 6-(Cyclopropylcarboxamido)-N-ethoxy-4-((2-methoxy-3-(1H-1,2,4-triazol-3-yl)phenyl)amino)pyridazine-3-carboxamide (Compound 1)

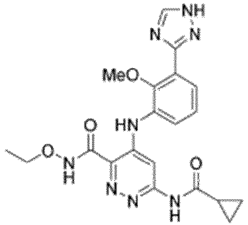

6-(Cyclopropanecarboxamido)-*N*-ethoxy-4-((2-methoxy-3-(1-(tetrahydro-2*H*-pyran-2-yl)-1*H*-1,2,4-triazol-3-yl)phenyl))amino)pyridazine-3-carboxamide (114 mg) was dissolved in methanol (2 mL), a hydrogen chloride dioxane solution (2 mL) was added thereto, and the mixture was allowed to react at room temperature for 1 hour. The reaction was stopped, the reaction solution was spun in vacuo to remove the solvent to obtain a crude product, and the crude product was separated and purified by reverse phase preparative chromatography (formic acid system) to obtain the title compound (12.71 mg, yield: 13.38%, white solid).

MS (ESI): m/z 439.1 [M+H]+.

1H NMR (400 MHz, DMSO-d6) δ 12.29 (s, 1H), 11.34 (s, 1H), 10.57 (s, 1H), 8.35 (s, 1H), 8.14 (s, 1H), 7.77 (d, J = 7.6 Hz, 1H), 7.56 (d, J = 6.6 Hz, 1H), 7.33 (t, J = 7.9 Hz, 1H), 3.99 (q, J = 7.0 Hz, 2H), 3.70 (s, 3H), 2.08 (t, J = 5.8 Hz, 1H), 1.23 (t, J = 7.0 Hz, 3H), 0.83-0.81 (m, 4H).

### Example 2

### 6-(Cyclopropylcarboxamido)-N-ethoxy-4-((2-methoxy-3-(1-(2-methoxyethyl)-1H-1,2,4-triazol-3-yl)phenyl)amino)pyridazine-3-carboxamide (Compound 2)

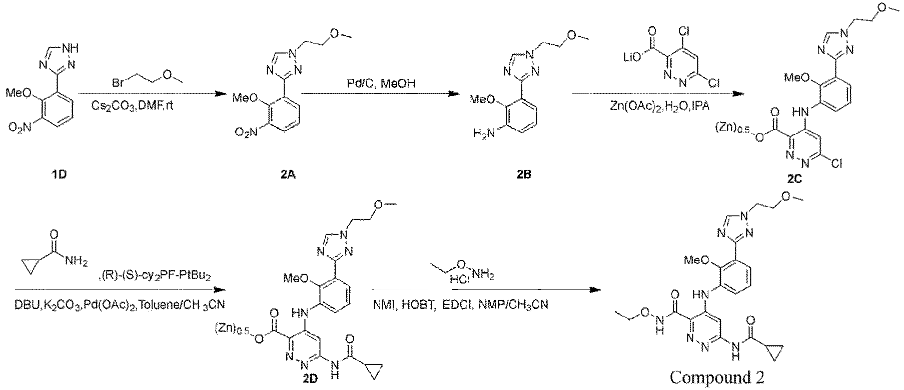

### Step 1: 3-(2-Methoxy-3-nitrophenyl)-1-(2-methoxyethyl)-1H-1,2,4-triazole

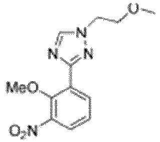

3-(2-Methoxy-3-nitrophenyl)-1*H*-1,2,4-triazole (1.0 g, 4.542 mmol) was dissolved in ultra-dry *N,N-*dimethylformamide (10 mL), and cesium carbonate (4439 mg, 13.626 mmol) was added. After replacement with nitrogen, 1-bromo-2-methoxyethane (947 mg, 6.813 mmol) was slowly added dropwise, and the mixture was allowed to react at room temperature for 2 hours. The reaction was stopped, and the reaction solution was filtered and then poured into water (30 mL). The aqueous phase was extracted using ethyl acetate (15 mL × 3), and the merged organic phase was washed with saturated brine (20 mL × 2), dried over anhydrous sodium sulfate and filtered, and the filtrate was spun in vacuo to remove the solvent to obtain the title compound (1.33 g, light yellow oily matter)

MS (ESI): m/z 279.0 [M+H]+.

### Step 2: 2-Methoxy-3-(1-(2-methoxyethyl)-1H-1,2,4-triazol-3-yl)aniline

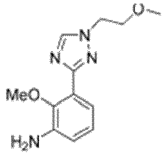

3-(2-Methoxy-3-nitrophenyl)-1-(2-methoxyethyl)-1*H*-1,2,4-triazole (1.3 g, 4.672 mmol) was dissolved in methanol (30 mL), and palladium/carbon (198 mg, 1.869 mmol) was added. After replacement with hydrogen three times, the mixture was allowed to react at room temperature for 16 hours. The reaction was stopped, and the reaction solution was filtered and then spun dry. A crude product was separated and purified by silica gel column chromatography (petroleum ether:ethyl acetate = 1:3) to obtain the title compound (830 mg, yield: 71.6%, colorless oily matter).

MS (ESI): m/z 249.1 [M+H]+.

### Step 3: ((6-Chloro-4-((2-methoxy-3-(1-(2-methoxyethyl)-1H-1,2,4-triazol-3-yl)phenyl)amino)pyridazine-3-carbonyl)oxy)zinc (0.5)

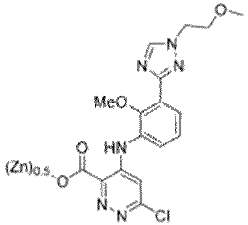

2-Methoxy-3-(1-(2-methoxyethyl)-1*H*-1,2,4-triazol-3-yl)aniline (750 mg, 3.021 mmol) was dissolved in water (21 mL) and isopropanol (3 mL), and lithium 4,6-dichloropyridazine-3-carboxylate (722 mg, 3.625 mmol) and zinc acetate (665 mg, 3.625 mmol) were added. After replacement with nitrogen three times, the mixture was allowed to react in an oil bath at 65°C for 16 hours. The reaction was stopped, and water (21 mL) was added to the reaction solution. The solution was stirred at room temperature for 1 hour, and then filtered by suction. The filter cake was rinsed using water (6 mL × 2) and tetrahydrofuran (1 mL), and dried to obtain the title compound (730 mg, yield: 55.3%, light yellow solid).

MS (ESI): m/z 404.9 [M+H]+.

### Step 4: ((6-(Cyclopropylcarboxamido)-4-((2-methoxy-3-(1-(2-methoxyethyl)-1H-1,2,4-triazol-3-yl)phenyl)amino)pyridazine-3-carbonyl)oxy)zinc (0.5)

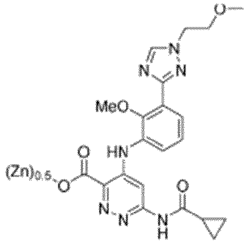

((6-Chloro-4-((2-methoxy-3-(1-(2-methoxyethyl)-1*H*-1,2,4-triazol-3-yl)phenyl)amino)pyridazine-3-carbonyl)oxy)zinc (0.5) (730 mg, 1.672 mmol) was dissolved in toluene (8 mL) and acetonitrile (4 mL), and cyclopropylcarboxamide (356 mg, 4.180 mmol), (*R*)-(-)-1-[(*S*)-2-(dicyclohexylphosphino)ferrocene]ethyl di-*tert*-butylphosphine (185 mg, 0.334 mmol), 1,8-diazabicycloundec-7-ene (254 mg, 1.672 mmol), potassium carbonate (462 mg, 3.344 mmol) and palladium acetate (75 mg, 0.334 mmol) were added. After replacement with nitrogen, the mixture was allowed to react in an oil bath at 75°C for 16 hours. The reaction was stopped, and the reaction solution was cooled to room temperature. Water (15 mL) and acetic acid (7.5 mL) were added, and the mixed system was washed using petroleum ether (30 mL × 2). The aqueous phase was extracted using dichloromethane (15 mL × 3), and the merged dichloromethane organic phase was dried over anhydrous sodium sulfate and filtered. The filtrate was spun in vacuo to remove the solvent to obtain the title compound (1410 mg, brown solid), which was directly used for the next step.

MS (ESI): m/z 454.6 [M+H]+.

### Step 5: 6-(Cyclopropylcarboxamido)-N-ethoxy-4-((2-methoxy-3-(1-(2-methoxyethyl)-1H-1,2,4-triazol-3-yl)phenyl)amino)pyridazine-3-carboxamide (Compound 2)

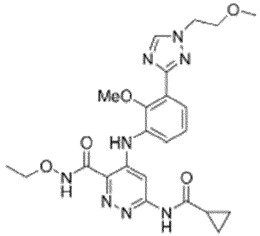

A 50 mL reaction flask was taken, into which *N*-methylpyrrolidone (3 mL) and acetonitrile (3 mL) were added. The mixed solvent was stirred at room temperature for 10 minutes, and then ((6-(cyclopropylcarboxamido)-4-((2-methoxy-3-(1-(2-methoxyethyl)-1*H*-1,2,4-triazol-3-yl)phenyl)amino)pyridazine-3-carbonyl)oxy)zinc (0.5) (400 mg, 0.825 mmol), *O-*ethylhydroxylamine hydrochloride (121 mg, 1.238 mmol) and *N-*methylimidazole (202 mg, 2.475 mmol) were added. The reaction solution was stirred in an oil bath at 65°C for 15 minutes, and then 1-hydroxybenzotriazole (223 mg, 1.650 mmol) and 1-ethyl-(3-dimethylaminopropyl)carbodiimide hydrochloride (316 mg, 1.650 mmol) were added. The reaction solution continued to be stirred and allowed to react in an oil bath at 65°C for 16 hours. The reaction was stopped, and the reaction solution was filtered. A crude product of the filtrate was separated and purified by high-performance liquid chromatography (elution system: formic acid, water and acetonitrile) to obtain the title compound (79.51 mg, yield: 19.4%, white solid).

MS (ESI): m/z 497.3 [M+H]+.

1H NMR (400 MHz, DMSO-d6) δ 12.25 (s, 1H), 11.31 (s, 1H), 10.55 (s, 1H), 8.57 (s, 1H), 8.15 (s, 1H), 7.66 (dd, J = 7.8, 1.5 Hz, 1H), 7.50 (dd, J = 7.9, 1.4 Hz, 1H), 7.27 (t, J = 7.9 Hz, 1H), 4.40 (t, J = 5.2 Hz, 2H), 3.98 (q, J = 7.0 Hz, 2H), 3.78-3.69 (m, 5H), 3.25 (s, 3H), 2.13-2.02 (m, 1H), 1.22 (t, J = 7.0 Hz, 3H), 0.81 (d, J = 5.4 Hz, 4H).

### Example 3

### 6-(Cyclopropylcarboxamido)-N-cyclopropyloxy-4-((2-methoxy-3-(1-(2-methoxyethyl)-1H-1,2,4-triazol-3-yl)phenyl)amino)pyridazine-3-carboxamide (Compound 3)

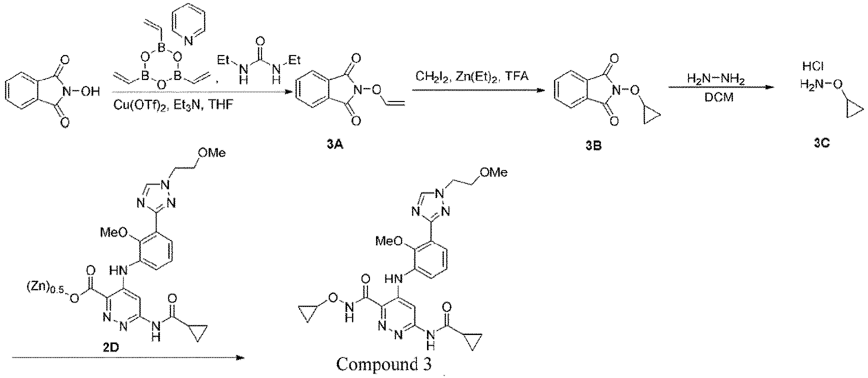

### Step 1: 2-(Vinyl)isoindoline-1,3-dione

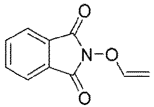

2-Hydroxyisoindoline-1,3-dione (900 mg, 5.517 mmol) was dissolved in ultra-dry tetrahydrofuran (20 mL), and an ethylene boric anhydride pyridine complex (876 mg, 3.641 mmol), diethyl urea (256 mg, 2.207 mmol), bis(((trifluoromethyl)sulfonyl)oxy)copper (798 mg, 2.207 mmol) and triethylamine (1114 mg, 11.034 mmol) were added. After replacement with oxygen, the mixture was allowed to react at 50°C for 16 hours. The reaction was stopped, the reaction solution was filtered and then the filtrate was collected and spun dry, and a crude product was separated and purified by silica gel column chromatography (petroleum ether: ethyl acetate = 4:1) to obtain the title compound (950 mg, yield: 91.1%, white solid).

1H NMR (400 MHz, DMSO-d6) δ 7.92-7.86 (m, 4H), 6.92 (dd, J = 13.6, 6.4 Hz, 1H), 4.73 (dd, J = 13.6, 3.5 Hz, 1H), 4.40 (dd, J = 6.4, 3.5 Hz, 1H).

### Step 2: 2-Cyclopropyloxyisoindoline-1,3-dione

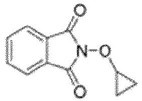

Diethylzinc (19 mL, 19.0 mmol, 1.0 M solution in hexanes) was added dropwise into ultra-dry dichloromethane (16 mL) at 0°C. After replacement with nitrogen, the temperature was kept at 0°C. Trifluoroacetic acid (2.172 g, 19.048 mmol) was dissolved in ultra-dry dichloromethane (8 mL) and then the resulting solution was slowly added dropwise into the above solution and stirring was performed for 20 minutes. Diiodomethane (5.101 g, 19.048 mmol) was dissolved in ultra-dry dichloromethane (8 mL) and then the resulting solution was slowly added dropwise, and stirring was continued for 20 minutes. 2-(Vinyl)isoindoline-1,3-dione (900 mg, 4.762 mmol) was dissolved in an ultra-dry dichloromethane solution (5.5 mL) and then the resulting solution was slowly added dropwise. After replacement with nitrogen, the mixture was allowed to react at room temperature for 16 hours. The reaction was stopped, and dilute hydrochloric acid (0.1 N, 16 mL) was added to the reaction solution, which was separated. The organic phase was collected and then washed using a saturated sodium bicarbonate solution (17 mL) and saturated brine (10 mL) in sequence, dried over anhydrous sodium sulfate, filtered, and spun dry to obtain a crude product. The crude product was separated and purified by silica gel column chromatography (petroleum ether: ethyl acetate = 5:1) to obtain the title compound (850 mg, yield: 87.9%, white solid).

### Step 3: O-Cyclopropylhydroxylamine hydrochloride

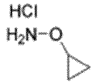

2-Cyclopropyloxyisoindoline-1,3-dione (450 mg, 2.217 mmol) was dissolved in dichloromethane (20 mL), and hydrazine hydrate (222 mg, 4.434 mmol) was added. After replacement with nitrogen, the mixture was allowed to react at room temperature for 16 hours. The reaction was stopped, the reaction solution was filtered, and the filtrate was washed using water (20 mL × 2). The aqueous phase was collected, and extraction was performed using a mixed chloroform/isopropanol solution (3:1, 15 mL × 6). The organic phases were merged and then dried, and a solution of hydrogen chloride in 1,4-dioxane (1 mL) was added. The resulting solution was stirred for 1 h and then spun dry to obtain the title compound (130 mg, yield: 53.5%, white solid).

### Step 4: 6-(Cyclopropylcarboxamido)-N-cyclopropyloxy-4-((2-methoxy-3-(1-(2-methoxyethyl)-1H-1,2,4-triazol-3-yl)phenyl)amino)pyridazine-3-carboxamide (Compound 3)

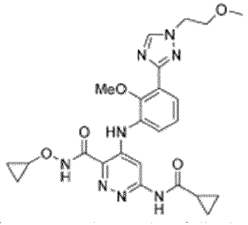

N-Methylpyrrolidone (3 mL) and acetonitrile (3 mL) were added to a reaction flask, and then ((6-(cyclopropylcarboxamido)-4-((2-(methoxy-3-(1-(2-methoxyethyl)-1*H*-1,2,4-triazol-3-yl)phenyl)amino)pyridazine-3-carbonyl)oxy)zinc (0.5) (400 mg, 0.825 mmol), *O-*cyclopropylhydroxylamine hydrochloride (136 mg, 1.238 mmol) and *N-*methylimidazole (202 mg, 2.475 mmol) were added. The reaction solution was stirred in an oil bath at 65°C for 15 minutes, and then 1-hydroxybenzotriazole (223 mg, 1.650 mmol) and 1-ethyl-(3-dimethylaminopropyl)carbodiimide hydrochloride (316 mg, 1.650 mmol) were added. The reaction solution was stirred and allowed to react in an oil bath at 65°C for 16 hours. The reaction was stopped, and the reaction solution was filtered. A crude product of the filtrate was separated and purified by high-performance liquid chromatography (elution system: formic acid, water and acetonitrile) to obtain the title compound (57 mg, yield: 13.6%, white solid).

MS (ESI): m/z 509.2 [M+H]+.

1H NMR (400 MHz, DMSO-d6) δ 12.46 (s, 1H), 11.33 (s, 1H), 10.56 (s, 1H), 8.58 (s, 1H), 8.17 (s, 1H), 7.68 (dd, J = 7.8, 1.5 Hz, 1H), 7.52 (dd, J = 8.0, 1.5 Hz, 1H), 7.30-7.26 (m, 1H), 4.42 (t, J = 5.2 Hz, 2H), 4.13-4.06 (m, 1H), 3.78-3.71 (m, 5H), 3.26 (s, 3H), 2.14-2.05 (m, 1H), 0.90 (brs, 2H), 0.82 (d, J = 5.4 Hz, 4H), 0.62-0.55 (m, 2H).

### Example 4

### 6-(Cyclopropylcarboxamido)-N-ethoxy-4-((3-(1-(2-fluoroethyl)-1H-1,2,4-triazol-3-yl)-2-methoxyphenyl)amino)pyridazine-3-carboxamide (Compound 4)

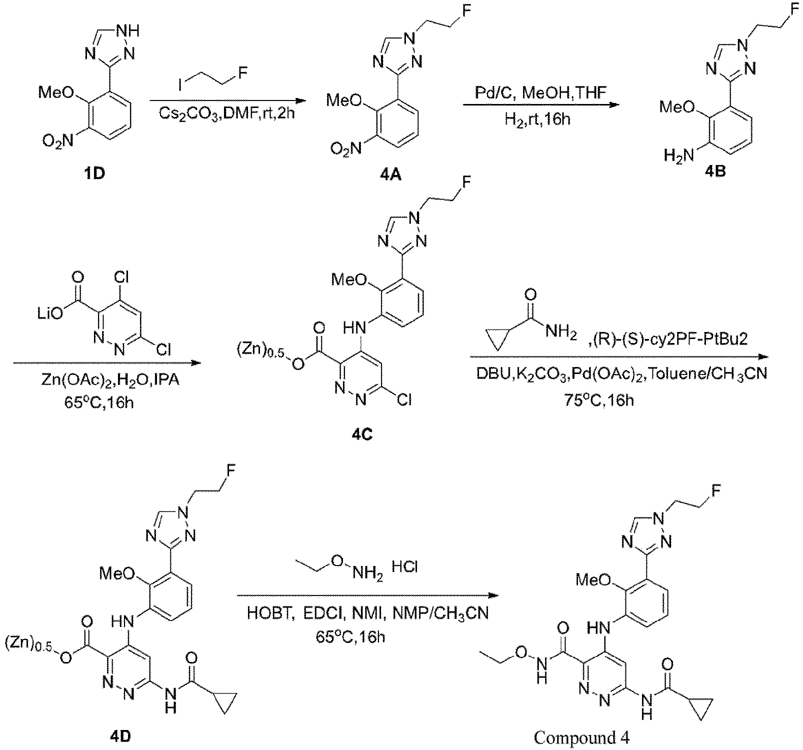

### Step 1: 1-(2-Fluoroethyl)-3-(2-methoxy-3-nitrophenyl)-1H-1,2,4-triazole

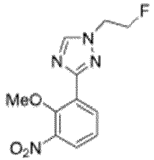

3-(2-Methoxy-3-nitrophenyl)-1*H*-1,2,4-triazole (1 g, 4.54 mmol), 1-fluoro-2-iodoethane (1.2 g, 6.81 mmol) and cesium carbonate (4.44 g, 13.62 mmol) were dissolved in *N,N-*dimethylformamide (10 mL), and the reaction solution was stirred and allowed to react at room temperature for 2 hours. The reaction was stopped, water (10 mL) was added to the reaction solution for quenching, and then extraction was performed using ethyl acetate (20 mL × 3). The merged organic phase was washed with saturated brine, dried over anhydrous sodium sulfate and filtered, and then the filtrate was concentrated under reduced pressure and then separated and purified by column chromatography (silica gel, dichloromethane:methanol = 97:3) to obtain the title compound (1.16 g, yield: 91.37%, yellow solid).

MS (ESI): m/z 267.0 [M+H]+.

### Step 2: 3-(1-(2-Fluoroethyl)-1H-1,2,4-triazol-3-yl)-2-methoxyaniline

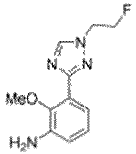

1-(2-Fluoroethyl)-3-(2-methoxy-3-nitrophenyl)-1*H*-1,2,4-triazole (1.11 g, 4.17 mmol) was dissolved in tetrahydrofuran (12 mL), methanol (20 mL) and aqueous ammonia (0.6 mL) were added, and then palladium/carbon (177.48 mg, 1.67 mmol) was added in batches. After replacement with hydrogen, the mixture was allowed to react at room temperature for 16 hours. The reaction was stopped. The reaction solution was filtered and concentrated under reduced pressure, and then separated and purified by column chromatography (silica gel, dichloromethane: methanol = 98:2) to obtain the title compound (728 mg, yield: 70.75%, white solid).

MS (ESI): m/z 237.1 [M+H]+.

### Step 3: ((6-Chloro-4-((3-(1-(2-fluoroethyl)-1H-1,2,4-triazol-3-yl)-2-methoxyphenyl)amino)pyridazine-3-carbonyl)oxy)zinc

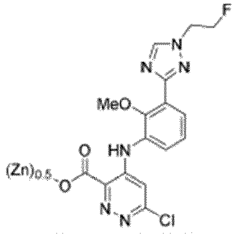

3-(1-(2-Fluoroethyl)-1*H*-1,2,4-triazol-3-yl)-2-methoxyaniline (300 mg, 1.27 mmol), lithium 4,6-dichloropyridazine-3-carboxylate (303.24 mg, 1.52 mmol) and zinc acetate (279.62 mg, 1.52 mmol) were dissolved in a 9 mL solvent of water: toluene = 7:1. After replacement with nitrogen, the mixture was allowed to react at 65°C for 16 hours. The reaction was stopped, 12 mL of water was added to the reaction solution, and the mixture was stirred for one hour and then filtered by suction and washed using tetrahydrofuran (0.5 mL). The filter cake was dried to obtain the title compound (360 mg, yield: 66.8%, white solid).

MS (ESI): m/z 393.0 [M+H]+.

### Step 4: ((6-(Cyclopropylcarboxamido)-4-((3-(1-(2-fluoroethyl)-1H-1,2,4-triazol-3-yl)-2-methoxyphenyl)amino)pyridazine-3-carbonyl)oxy)zinc (0.5)

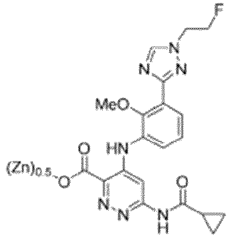

((6-Chloro-4-((3-(1-(2-fluoroethyl)-1*H*-1,2,4-triazol-3-yl)-2-methoxyphenyl)amino)pyridazine-3-carbonyl)oxy)zinc (0.5) (360 mg, 0.848 mmol), cyclopropylcarboxamide (216.53 mg, 2.544 mmol), (2*R*)-1-[(1*R*)-1-[bis(1,1-di-*tert-*butyl)phosphino]ethyl]-2-(dicyclohexylphosphino)ferrocene (94.07 mg, 0.17 mmol), 1,8-diazabicyclo[5.4.0]undec-7-ene (129.12 mg, 0.848 mmol), potassium carbonate (351.66 mg, 2.544 mmol) and palladium acetate (38.08 mg, 0.17 mmol) were dissolved in 6 mL of a solvent of toluene:acetonitrile = 2:1. After replacement with nitrogen, the mixture was allowed to react at 75°C for 16 hours. The reaction was stopped, and a 12 mL solution of water: acetic acid = 2:1 was added to the reaction solution, which was washed using petroleum ether (20 mL). The aqueous phase was extracted using dichloromethane (20 mL × 3), and the merged dichloromethane organic phase was washed with saturated brine, dried over anhydrous sodium sulfate and filtered, and then the filtrate was spun in vacuo to remove the solvent to obtain the title compound (382 mg, yield: 95.2%, brown solid).

MS (ESI): m/z 442.2 [M+H]+.

### Step 5: 6-(Cyclopropylcarboxamido)-N-ethoxy-4-((3-(1-(2-fluoroethyl)-1H-1,2,4-triazol-3-yl)-2-methoxyphenyl)amino)pyridazine-3-carboxamide (Compound 4)

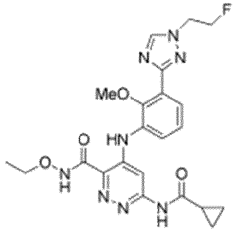

*N*-Methylpyrrolidone (11.4 mL) and acetonitrile (11.4 mL) were added to a reaction flask, and bis((6-(cyclopropanecarboxamido)-4-((3-(1-(2-fluoroethyl)-1*H*-1,2,4-triazol-3-yl)-2-methoxyphenyl)amino)pyridazine-3-carbonyl)oxy)zinc (380 mg, 0.803 mmol), *O-*ethylhydroxylamine hydrochloride (117.52 mg, 1.205mmol) and *N*-methylimidazole (197.83mg, 2.41mmol) were added. After replacement with nitrogen, the mixture was allowed to react in an oil bath at 65°C for 15 minutes, and finally 1-hydroxybenzotriazole (217.06 mg, 1.61 mmol) and 1-(3-dimethylaminopropyl)-3-ethylcarbodiimide hydrochloride (307.95 mg, 1.61 mmol) were added. After replacement with nitrogen, the mixture then continued to react in an oil bath at 65°C for 16 hours. The reaction was stopped, water (20 mL) was added to the reaction solution for quenching, then extraction was performed using ethyl acetate (25 mL × 3), and the merged organic phase was washed with saturated brine, dried over anhydrous sodium sulfate, and spun in vacuo to remove the solvent to obtain a crude product. The crude product was separated and purified by reverse phase preparative chromatography (elution system: formic acid, water and acetonitrile) to obtain the title compound (29.6 mg, yield: 7.6%, white solid).

MS (ESI): m/z 485.2 [M+H]+.

1H NMR (400 MHz, DMSO) δ12.22 (s, 1H), 11.33 (s, 1H), 10.57 (s, 1H), 8.66 (s, 1H), 8.16 (s, 1H), 7.69 (d, J= 7.7 Hz, 1H), 7.53 (d, J = 7.8 Hz, 1H), 7.29 (t, J = 7.9 Hz, 1H), 4.91 (t, J = 4.6 Hz, 1H), 4.79 (t, J = 4.6 Hz, 1H), 4.64 (t, J = 4.6 Hz, 1H), 4.57 (t, J = 4.6 Hz, 1H), 3.99 (q, J = 7.0 Hz, 2H), 3.73 (s, 3H), 2.15-2.01 (m, 1H), 1.23 (t, J = 7.0 Hz, 3H), 0.82 (d, J = 5.4 Hz, 4H).

### Example 5

### 6-(Cyclopropylcarboxamido)-4-((3-(1-(2,2-difluoroethyl)-1H-1,2,4-triazol-3-yl)-2-methoxyphenyl)amino)-N-ethoxypyridazine-3-carboxamide (Compound 5)

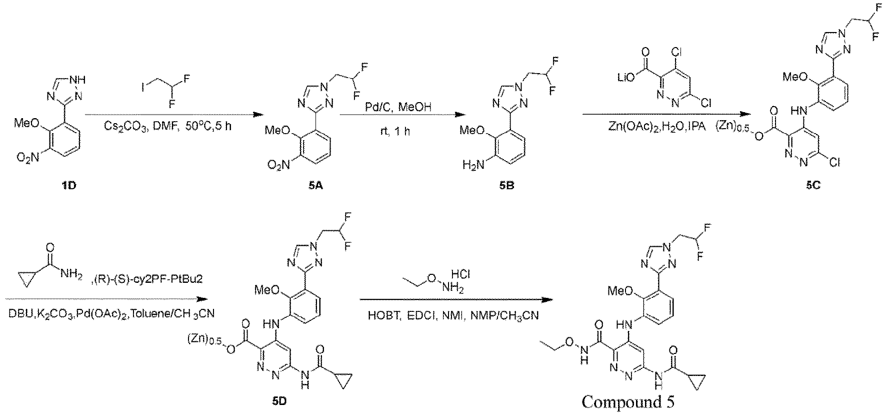

### Step 1: 1-(2,2-Difluoroethyl)-3-(2-methoxy-3-nitrophenyl)-1H-1,2,4-triazole

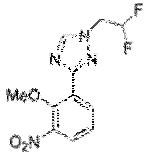

3-(2-Methoxy-3-nitrophenyl)-1*H*-1,2,4-triazole (900 mg, 4.087 mmol) was dissolved in *N,N*-dimethylformamide (8 mL), cesium carbonate (3.995 g, 12.261 mmol) was added, and then 1,1-difluoro-2-iodoethane (1.177 g, 6.131 mmol) was added at 0°C. After replacement with nitrogen, the mixture was allowed to react at 65°C for 5 hours. The reaction was stopped, and the reaction solution was filtered and then water (20 mL) was added thereto, followed by extraction using ethyl acetate (10 mL × 3). The organic phases were merged and then washed using saturated brine (10 mL × 2), dried over anhydrous sodium sulfate and filtered, and then the filtrate was spun dry to obtain the title compound (1.05 g, yield: 90.3%, light yellow oily matter).

MS (ESI): m/z 285.2 [M+H]+.

### Step 2: 3-(1-(2,2-Difluoroethyl)-1H-1,2,4-triazol-3-yl)-2-methoxyaniline

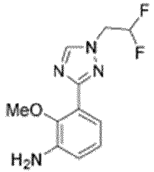

1-(2,2-Difluoroethyl)-3-(2-methoxy-3-nitrophenyl)-1*H*-1,2,4-triazole (950 mg, 3.339 mmol) was dissolved in MeOH (20 mL), and palladium/carbon (146 mg, 1.336 mmol) was added. After replacement with hydrogen, the mixture was allowed to react at room temperature for 2 hours. The reaction was stopped, and the reaction solution was filtered and then spun dry. A crude product was separated and purified by silica gel column chromatography (petroleum ether:ethyl acetate = 1:1) to obtain the title compound (910 mg, yield: 83.6%, light yellow oily matter).

MS (ESI): m/z 255.0 [M+H]+.

### Step 3 : ((6-Chloro-4-((3-(1-(2,2-difluoroethyl)-1H-1,2,4-triazol-3-yl)-2-methoxyphenyl)amino)pyridazine-3-carbonyl)oxy)zinc (0.5)

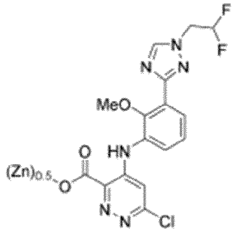

3-(1-(2,2-Difluoroethyl)-1*H*-1,2,4-triazol-3-yl)-2-methoxyaniline (830 mg, 3.264 mmol) was dissolved in water (21 mL) and isopropanol (3 mL), and lithium 4,6-dichloropyridazine-3-carboxylate (779 mg, 3.917 mmol) and zinc acetate (719 mg, 3.917 mmol) were added. After replacement with nitrogen three times, the mixture was allowed to react in an oil bath at 65°C for 16 hours. The reaction was stopped, and water (25 mL) was added to the reaction solution. The solution was stirred at room temperature for 1 hour, and then filtered by suction. The filter cake was rinsed using water (5 mL × 2) and tetrahydrofuran (1 mL), and dried to obtain the title compound (940 mg, yield: 65.1%, white solid).

MS (ESI): m/z 411.0 [M+H]+.

### Step 4: ((6-(Cyclopropylcarboxamido)-4-((3-(1-(2,2-difluoroethyl)-1H-1,2,4-triazol-3-yl)-2-methoxyphenyl)amino)pyridazine-3-carbonyl)oxy)zinc (0.5)

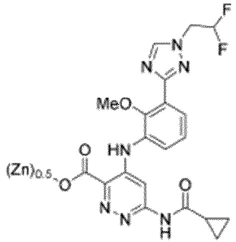

((6-Chloro-4-((3-(1-(2,2-difluoroethyl)-1*H*-1,2,4-triazol-3-yl)-2-methoxyphenyl)amino)pyridazine-3-carbonyl)oxy)zinc (0.5) (420 mg, 0.949 mmol) was dissolved in toluene (4 mL) and acetonitrile (2 mL), and then cyclopropylcarboxamide (202 mg, 2.373 mmol), potassium carbonate (262 mg, 1.898 mmol), 1,8-diazacyclo[5.4.0]undec-7-ene (144 mg, 0.949 mmol), (*R*)-(-)-1-[(*S*)*-*2*-*(dicyclohexylphosphino)ferrocene]ethyl di-*tert*-butylphosphine (105 mg, 0.190 mmol) and palladium acetate (43 mg, 0.190 mmol) were added. After replacement with nitrogen, the mixture was allowed to react at 75°C for 16 hours. The reaction was stopped, and water (15 mL) and acetic acid (7.5 mL) were added to the reaction solution, which was washed using petroleum ether (30 mL × 2), and the aqueous phase was then extracted using dichloromethane (10 mL × 3). The dichloromethanes were merged and dried over anhydrous sodium sulfate. The filtrate was filtered and spun dry to obtain the crude title compound (520 mg, brown oily matter) which was directly used for the next step.

MS (ESI): m/z 460.0 [M+H]+.

### Step 5: 6-(Cyclopropylcarboxamido)-4-((3-(1-(2,2-difluoroethyl)-1H-1,2,4-triazol-3-yl)-2-methoxyphenyl)amino)-N-ethoxypyridazine-3-carboxamide (Compound 5)

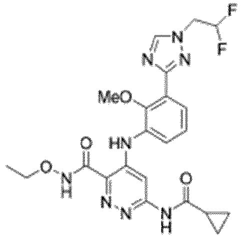

*N*-Methylpyrrolidone (3 mL) and acetonitrile (3 mL) were added to a reaction flask, and then ((6-(cyclopropylcarboxamido)-4-((3-(1-(2,2-difluoroethyl)-1*H*-1,2,4-triazol-3-yl)-2-methoxyphenyl)amino)pyridazine-3-carbonyl)oxy)zinc (0.5) (470 mg, 0.957 mmol), *O-*ethylhydroxylamine hydrochloride (140 mg, 1.436 mmol) and *N-*methylimidazole (235 mg, 2.871 mmol) were added. The reaction solution was stirred in an oil bath at 65°C for 15 minutes, and then 1-hydroxybenzotriazole (259 mg, 1.914 mmol) and 1-ethyl-(3-dimethylaminopropyl)carbodiimide hydrochloride (367 mg, 1.914 mmol) were added. The reaction solution was stirred and allowed to react in an oil bath at 65°C for 5 hours. The reaction was stopped, and the reaction solution was filtered. A crude product of the filtrate was separated and purified by high-performance liquid chromatography (elution system: trifluoroacetic acid, water and acetonitrile) to obtain the title compound (13.54 mg, yield: 2.8%, white solid).

MS (ESI): m/z 503.0 [M+H]+.

1H NMR (400 MHz, DMSO-d6) δ 12.27 (s, 1H), 11.33 (s, 1H), 10.57 (s, 1H), 8.69 (s, 1H), 8.16 (s, 1H), 7.68 (dd, J = 7.8, 1.3 Hz, 1H), 7.54 (d, J = 6.7 Hz, 1H), 7.29 (t, J = 7.9 Hz, 1H), 6.64-6.32 (m, 1H), 4.83 (td, J = 15.2, 3.4 Hz, 2H), 3.99 (q, J = 7.0 Hz, 2H), 3.72 (s, 3H), 2.12-2.04 (m, 1H), 1.23 (t, J = 7.0 Hz, 3H), 0.82 (d, J = 4.6 Hz, 4H).

### Example 6

### 6-(Cyclopropylcarboxamido)-N-cyclopropyloxy-4-((3-(1-(2-fluoroethyl)-1H-1,2,4-triazol-3-yl)-2-methoxyphenyl)amino)pyridazine-3-carboxamide (Compound 6)

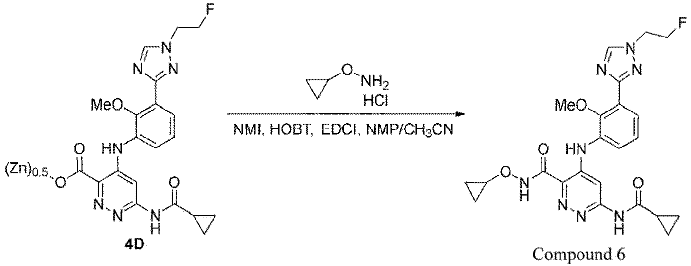

*N*-Methylpyrrolidone (4 mL) and acetonitrile (4 mL) were added to a reaction flask, and ((6-(cyclopropylcarboxamido)-4-((3-(1-(2-fluoroethyl)-1*H*-1,2,4-triazol-3-yl)-2-methoxyphenyl)amino)pyridazine-3-carbonyl)oxy)zinc (830 mg, 1.754 mmol), *O-*cyclopropylhydroxylamine hydrochloride (288 mg, 2.631 mmol) and *N-*methylimidazole (430 mg, 5.262 mmol) were added. The reaction solution was stirred in an oil bath at 65°C for 15 minutes, and then 1-hydroxybenzotriazole (474 mg, 3.508 mmol) and 1-ethyl-(3-dimethylaminopropyl)carbodiimide hydrochloride (672 mg, 3.508 mmol) were added. The reaction solution was stirred and allowed to react in an oil bath at 65°C for 16 hours. The reaction was stopped, and the reaction solution was filtered. A crude product of the filtrate was separated and purified by high-performance liquid chromatography (elution system: formic acid, water and acetonitrile) to obtain the title compound (82.74 mg, yield: 63.3%, grey solid).

MS (ESI): m/z 497.3 [M+H]+.

1H NMR (400 MHz, DMSO-d6) δ12.33 (s, 1H), 11.32 (s, 1H), 10.55 (s, 1H), 8.65 (s, 1H), 8.16 (s, 1H), 7.68 (dd, J = 7.8, 1.4 Hz, 1H), 7.52 (dd, J =7.9, 1.3 Hz, 1H), 7.28 (t, J = 7.9 Hz, 1H), 4.90 (t, J = 4.6 Hz, 1H), 4.78 (t, J = 4.6 Hz, 1H), 4.63 (t, J = 4.6 Hz, 1H), 4.56 (t, J = 4.6 Hz, 1H), 4.09 (brs, 1H), 3.72 (s, 3H), 2.10-2.05 (m, 1H), 0.89 (brs, 2H), 0.81 (d, J = 5.3 Hz, 4H), 0.61-0.54 (m, 2H).

### Example 7

### 2-(3-(3-((6-(Cyclopropylcarboxamido)-3-(ethoxycarbamoyl)pyridazine-4-yl)amino)-2-methoxyphenyl)-1H-1,2,4-triazol-1-yl)acetic acid (Compound 7)

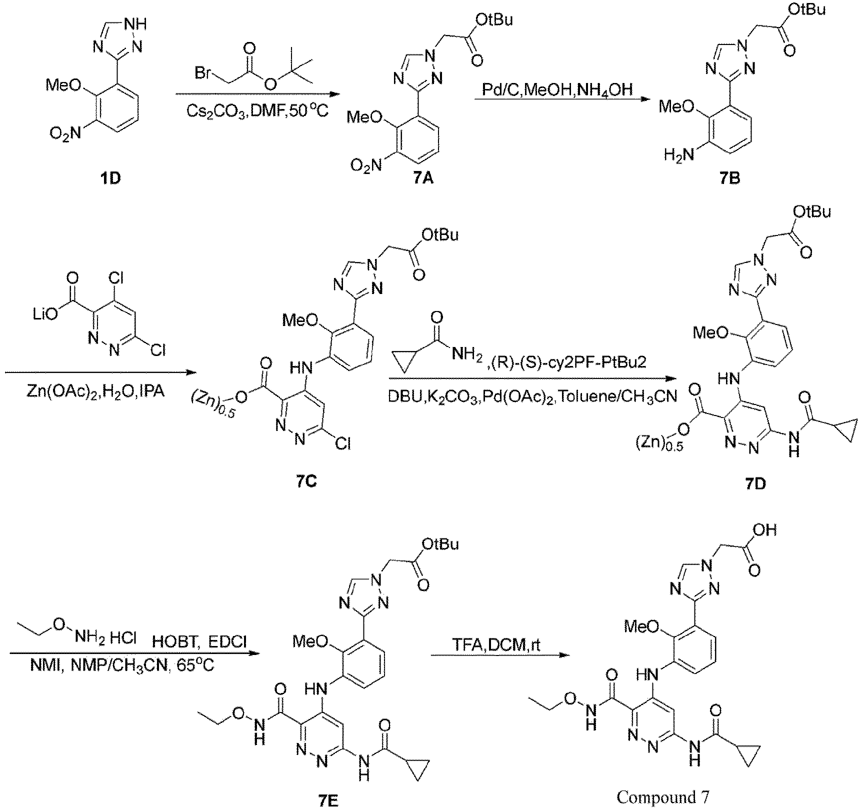

### Step 1: tert-Butyl 2-(3-(2-methoxy-3-nitrophenyl)-1H-1,2,4-triazol-1-yl)acetate

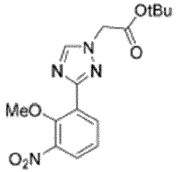

3-(2-Methoxy-3-nitrophenyl)-1*H*-1,2,4-triazole (550 mg, 2.50 mmol), *tert*-butyl 2-bromoacetate (730.8 mg, 3.75 mmol) and cesium carbonate (1.63 g, 5.00 mmol) were dissolved in *N,N*-dimethylformamide (5.5 mL), and the reaction solution was stirred and allowed to react at 50°C for 1 hour. The reaction was stopped, water (5 mL) was added to the reaction solution for quenching, and then extraction was performed using ethyl acetate (10 mL × 3). The merged organic phase was washed with saturated brine, dried over anhydrous sodium sulfate and filtered, and then the filtrate was concentrated under reduced pressure and then separated and purified by column chromatography (silica gel, petroleum ether:ethyl acetate = 4:1) to obtain the title compound (554 mg, yield: 66.34%, white solid).

MS (ESI): m/z 335.0 [M+H]+.

### Step 2: tert-Butyl 2-(3-(3-amino-2-methoxyphenyl)-1H-1,2,4-triazol-1-yl)acetate

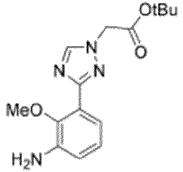

*tert*-Butyl 2-(3-(2-methoxy-3-nitrophenyl)-1*H*-1,2,4-triazol-1-yl)acetate (554 mg, 1.66 mmol) was dissolved in methanol (20 mL), ammonium hydroxide (0.3 mL) was added, and then palladium/carbon (70.54 mg, 0.66 mmol) was added in batches. After replacement with hydrogen, the mixture was allowed to react at room temperature for 16 hours. The reaction was stopped, the reaction solution was filtered, and the filtrate was concentrated under reduced pressure and then separated and purified by column chromatography (silica gel, petroleum ether: ethyl acetate = 3:7) to obtain the title compound (300 mg, yield: 59.49%, yellow solid).

MS (ESI): m/z 305.1 [M+H]+.

### Step 3: ((4-((3-(1-(2-(tert-Butyloxy)-2-oxoethyl)-1H-1,2,4-triazol-3-yl)-2-methoxyphenyl)amino)-6-chloropyridazine-3-carbonyl)oxy)zinc (0.5)

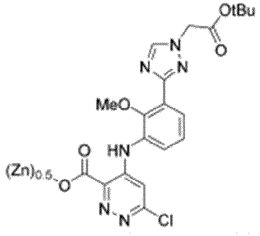

*tert*-Butyl 2-(3-(3-amino-2-methoxyphenyl)-1*H*-1,2,4-triazol-1-yl)acetate (200 mg, 0.657 mmol), lithium 4,6-dichloropyridazine-3-carboxylate (156.93 mg, 0.789 mmol) and zinc acetate (144.7 mg, 0.789 mmol) were dissolved in a 6 mL solvent of water:acetonitrile = 7:1. After replacement with nitrogen, the mixture was allowed to react in an oil bath at 65°C for 16 hours. The reaction was stopped, 8 mL of water was added to the reaction solution, and the mixture was stirred for one hour and then filtered by suction and washed using tetrahydrofuran (0.5 mL). The filter cake was dried to obtain the title compound (185 mg, yield: 57.4%, white solid).

MS (ESI): m/z 461.1 [M+H]+.

### Step 4: ((4-((3-(1-(2-(tert-Butoxy)-2-oxoethyl)-1H-1,2,4-triazol-3-yl)-2-methoxyphenyl)amino)-6-(cyclopropanecarboxamido)pyridazine-3-carbonyl)oxy)zinc (0.5)

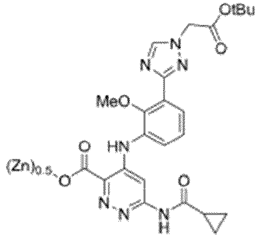

((4-((3-(1-(2-(*tert*-Butyloxy)-2-oxoethyl)-1*H*-1,2,4-triazol-3-yl)-2-methoxyphenyl)amino)-6-chloropyridazine-3-carbonyl)oxy)zinc (0.5) (140 mg, 0.285 mmol), cyclopropylcarboxamide (72.86 mg, 0.856 mmol), (2*R*)-1-[(1*R*)-1-[bis(1,1-di*-tert-*butyl)phosphino]ethyl]-2-(dicyclohexylphosphino)ferrocene (31.65 mg, 0.0571 mmol), 1,8-diazabicyclo[5.4.0]undec-7-ene (43.45 mg, 0.285 mmol), potassium carbonate (118.33 mg, 0.856 mmol) and palladium acetate (12.81 mg, 0.0571 mmol) were dissolved in a 2 mL solvent of toluene:acetonitrile = 2:1. After replacement with nitrogen, the mixture was allowed to react at 75°C for 16 hours. The reaction was stopped, and a 4.5 mL solution of water: acetic acid = 2:1 was added to the reaction solution, which was washed using petroleum ether (8 mL), and then extracted using dichloromethane (16 mL × 3). The merged dichloromethane organic phase was washed with saturated brine, dried over anhydrous sodium sulfate and filtered, and then the filtrate was spun in vacuo to remove the solvent to obtain the title compound (104 mg, yield: 67.3%, brown oily matter).

MS (ESI): m/z 510.1 [M+H]+.

### Step 5: tert-Butyl 2-(3-(3-((6-(cyclopropylcarboxamido)-3-(ethoxycarbamoyl)pyridazine-4-yl)amino)-2-methoxyphenyl)-1H-1,2,4-triazole-1-acetate

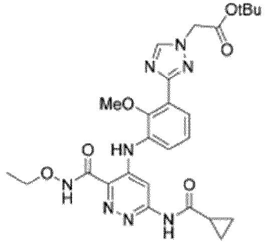

*N*-Methylpyrrolidone (2 mL) and acetonitrile (2 mL) were added to a reaction flask, and ((4-((3-(1-(2-(*tert*-butyloxy)-2-oxoethyl)-1*H*-1,2,4-triazol-3-yl)-2-methoxyphenyl)amino)-6-(cyclopropanecarboxamido)pyridazine-3-carbonyl)oxy)zinc (0.5) (84 mg, 0.155 mmol), *O-*ethylhydroxylamine hydrochloride (22.91 mg, 0.233 mmol) and *N*-methylimidazole (38.23 mg, 0.466 mmol) were added. After replacement with nitrogen, the mixture was allowed to react in an oil bath at 65°C for 15 minutes. Finally, 1-hydroxybenzotriazole (41.94 mg, 0.31 mmol) and 1-(3-dimethylaminopropyl)-3-ethylcarbodiimide hydrochloride (59.51 mg, 0.31 mmol) were added, and after replacement with nitrogen, the mixture was allowed to react in an oil bath at 65°C for 16 hours. The reaction was stopped, and the reaction solution was extracted using ethyl acetate (8 mL × 3). The merged organic phase was washed with saturated brine, dried over anhydrous sodium sulfate and filtered, and then the filtrate was dried in vacuo to remove the solvent to obtain the title compound (305 mg, purity: 73.73%, brown oily matter).

MS (ESI): m/z 553.1 [M+H]+.

### Step 6: 2-(3-(3-((6-(Cyclopropylcarboxamido)-3-(ethoxycarbamoyl)pyridazine-4-yl)amino)-2-methoxyphenyl)-1H-1,2,4-triazol-1-yl)acetic acid (Compound 7)

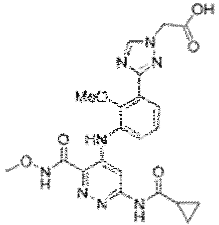

*tert*-Butyl 2-(3-(3-((6-(cyclopropylcarboxamido)-3-(ethoxycarbamoyl)pyridazine-4-yl)amino)-2-methoxyphenyl)-1*H*-1,2,4-triazole-1-acetate (300 mg) was dissolved in a 10 mL solvent of trifluoroacetic acid: 1,2-dichloroethane = 1:1 and was allowed to react at room temperature for 5 hours. The reaction was stopped, and the reaction solution was spun in vacuo to remove the solvent to obtain a crude product, and the crude product was separated and purified by reverse phase preparative chromatography to obtain the title compound (5 mg, yield: 1.8%, white solid).

MS (ESI): m/z 497.1 [M+H]+.

1H NMR (400 MHz, DMSO-d6) δ 11.32 (s, 1H), 10.55 (s, 1H), 8.49 (s, 1H), 8.16 (s, 1H), 7.67 (d, J = 7.7 Hz, 1H), 7.49 (d, J = 7.6 Hz, 1H), 7.26 (t, J = 7.9 Hz, 1H), 4.71 (s, 2H), 3.99 (q, J = 7.0 Hz, 2H), 3.71 (s, 3H), 2.14-2.01 (m, 1H), 1.23 (t, J = 7.0 Hz, 3H), 0.82 (d, J = 5.5 Hz, 4H).

### Example 8

### 3-(3-(3-((6-(Cyclopropylcarboxamido)-3-(ethoxycarbamoyl)pyridazine-4-yl)amino)-2-methoxyphenyl)-1H-1,2,4-triazol-1-yl)propanoic acid (Compound 8)

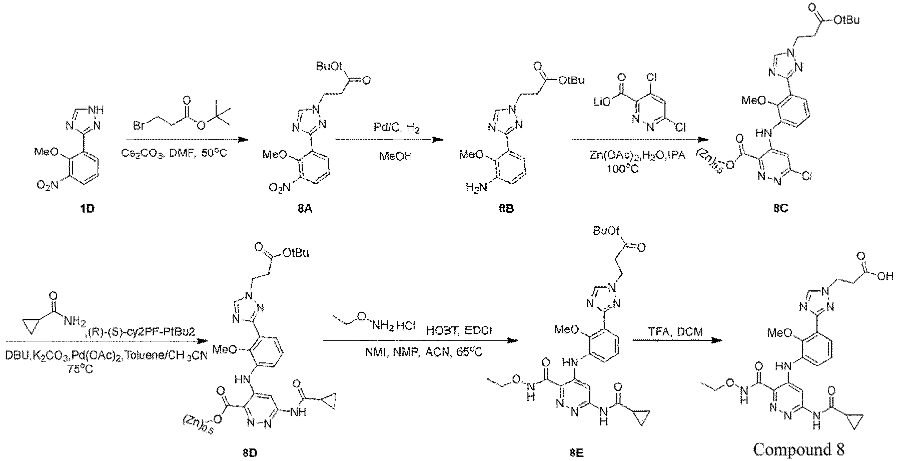

### Step 1: tert-Butyl 3-(3-(2-methoxy-3-nitrophenyl)-1H-1,2,4-triazol-1-yl)propanoate

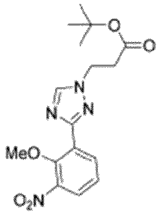

3-(2-Methoxy-3-nitrophenyl)-1*H*-1,2,4-triazole (400 mg, 1.818 mmol) was dissolved in ultra-dry *N,N-*dimethylformamide (5 mL), and cesium carbonate (1.78 g, 5.454 mmol) was added. After replacement with nitrogen, *tert*-butyl 3-bromopropionate (570 mg, 2.727 mmol) was slowly added dropwise in an ice bath at 0°C, and then the mixture was allowed to react at 50°C for 16 hours. The reaction was stopped, and the reaction solution was poured into water (20 mL). The aqueous phase was extracted using ethyl acetate (10 mL × 3), and the merged organic phase was washed with saturated brine (15 mL × 3), dried over anhydrous sodium sulfate and filtered, and then the filtrate was spun in vacuo to remove the solvent to obtain a crude product. The crude product was separated and purified by silica gel column chromatography (dichloromethane:methanol = 20:1) to obtain the title compound (490 mg, yield: 77.5%, light yellow oily matter)

MS (ESI): m/z 349.0 [M+H]+.

### Step 2: tert-Butyl 3-(3-(3-amino-2-methoxyphenyl)-1H-1,2,4-triazol-1-yl)propanoate

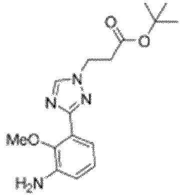

*tert*-Butyl 3-(3-(2-methoxy-3-nitrophenyl)-1*H*-1,2,4-triazol-1-yl)propanoate (490 mg, 1.408 mmol) was dissolved in methanol (20 mL), and a palladium/carbon catalyst (37 mg, 0.352 mmol) was added. After replacement with hydrogen three times, the mixture was allowed to react at room temperature for 2 hours. The reaction was stopped, and filtering was performed to remove the palladium/carbon. The filtrate was spun in vacuo to remove the solvent to obtain a crude product, and the crude product was separated and purified by silica gel column chromatography (dichloromethane:methanol = 10:1) to obtain the title compound (420 mg, yield: 93.8%, light yellow oily matter)

MS (ESI): m/z 319.4 [M+H]+.

### Step 3: ((4-((3-(1-(3-(tert-Butyloxy)-3-oxopropyl)-1H-1,2,4-triazol-3-yl)-2-methoxyphenyl)amino)-6-chloropyridazine-3-carbonyl)oxy)zinc (0.5)

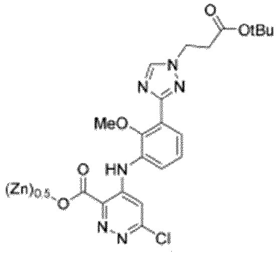

*tert*-Butyl 3-(3-(3-amino-2-methoxyphenyl)-1*H*-1,2,4-triazol-1-yl)propanoate (420 mg, 1.321 mmol) was dissolved in water (7 mL) and isopropanol (1 mL), and lithium 4,6-dichloropyridazine-3-carboxylate (304 mg, 1.585 mmol) and zinc acetate (291 mg, 1.585 mmol) were added. After replacement with nitrogen three times, the mixture was allowed to react in an oil bath at 65°C for 16 hours. The reaction was stopped, and water (10 mL) was added to the reaction solution. The solution was stirred at room temperature for 1 hour, and then filtered by suction. The filter cake was rinsed using water (6 mL × 2) and tetrahydrofuran (1 mL), and dried to obtain the title compound (320 mg, yield: 47.9%, brown solid).

MS (ESI): m/z 475.1 [M+H]+.

### Step 4: ((4-((3-(1-(3-(tert-Butyloxy)-3-oxopropyl)-1H-1,2,4-triazol-3-yl)-2-methoxyphenyl)amino)-6-(cyclopropylcarboxamido)pyridazine-3-carbonyl)oxy)zinc (0.5)

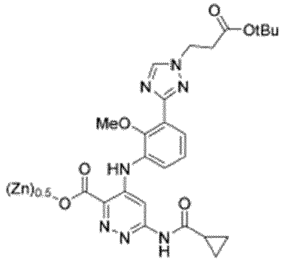

((4-((3-(1-(3-(*tert*-Butoxy)-3-oxopropyl)-1*H*-1,2,4-triazol-3-yl)-2-methoxyphenyl)amino)-6-chloropyridazine-3-carbonyl)oxy)zinc (0.5) (320 mg, 0.632 mmol) was dissolved in toluene (4 mL) and acetonitrile (2 mL), and cyclopropylcarboxamide (134 mg, 1.580 mmol), (*R*)-(-)-1-[(*S*)-2-(dicyclohexylphosphino)ferrocene]ethyl di-*tert*-butylphosphine (70 mg, 0.126 mmol), 1,8-diazabicycloundec-7-ene (96 mg, 0.632 mmol), cesium carbonate (174 mg, 1.264 mmol) and palladium acetate (28 mg, 0.126 mmol) were added. After replacement with nitrogen, the mixture was allowed to react in an oil bath at 100°C for 16 hours. The reaction was stopped, and the reaction solution was cooled to room temperature. Water (8 mL) and acetic acid (4 mL) were added, and the mixed system was washed using petroleum ether (15 mL × 2). The aqueous phase was extracted using dichloromethane (20 mL × 3), and the merged dichloromethane organic phase was washed with saturated brine (10 mL × 2), dried over anhydrous sodium sulfate and filtered, and then the filtrate was spun in vacuo to remove the solvent to obtain the title compound (275 mg, yield: 78.4%, brown solid).

MS (ESI): m/z 524.2 [M+H]+.

### Step 5: tert-Butyl 3-(3-(6-(cyclopropylcarboxamido)-3-(ethoxycarbamoyl)pyridazine-4-yl)amino)-2-methoxyphenyl)-1H-1,2,4-triazol-1-yl)propanoate

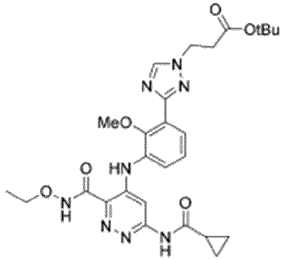

*N*-Methylpyrrolidone (2 mL) and acetonitrile (2 mL) were added to a reaction flask, and ((4-((3-(1-(3-(*tert*-butyloxy)-3-oxopropyl)-1*H*-1,2,4-triazol-3-yl)-2-methoxyphenyl)amino)-6-(cyclopropylcarboxamido)pyridazine-3-carbonyl)oxy)zinc (0.5) (275 mg, 0.495 mmol), *O-*ethylhydroxylamine hydrochloride (72 mg, 0.743 mmol) and N-methylimidazole (121 mg, 1.485 mmol) were added. The reaction solution was stirred in an oil bath at 65°C for 15 minutes, and then 1-hydroxybenzotriazole (134 mg, 0.990 mmol) and 1-ethyl-(3-dimethylaminopropyl)carbodiimide hydrochloride (190 mg, 0.990 mmol) were added. The reaction solution was stirred in an oil bath at 65°C for 16 hours. The reaction was stopped, and water (10 mL) was added to the reaction solution for quenching. The aqueous phase was extracted using ethyl acetate (10 mL × 3), and the merged organic phase was washed with saturated brine (10 mL × 2), dried over anhydrous sodium sulfate and filtered, and then the filtrate was spun in vacuo to remove the solvent to obtain the title compound (300 mg, yield: 8.02%, yellow solid).

MS (ESI): m/z 567.1 [M+H]+.

### Step 6: 3-(3-(3-((6-(Cyclopropylcarboxamido)-3-(ethoxycarbamoyl)pyridazine-4-yl)amino)-2-methoxyphenyl)-1H-1,2,4-triazol-1-yl)propanoic acid (Compound 8)

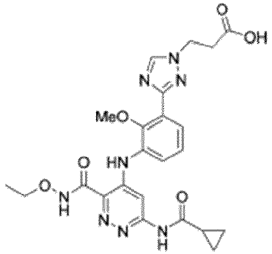

*tert*-Butyl 3-(3-(6-(cyclopropanecarboxamide)-3-(ethoxycarbamoyl)pyridazine-4-yl)amino)-2-methoxyphenyl)-1*H*-1,2,4-triazol-1-yl)propanoate 10 (300 mg, 0.531 mmol) was dissolved in dichloromethane (1 mL), trifluoroacetic acid (1 mL) was added, and the mixture was stirred at room temperature for 1 hour. The reaction was stopped, and the reaction solution was spun dry. A crude product was separated and purified by high-performance liquid chromatography (elution system: formic acid, water and acetonitrile) to obtain the title compound (37.98 mg, yield: 14.0%, light yellow solid).

MS (ESI): m/z 511.2 [M+H]+.

1H NMR (400 MHz, DMSO-d6) δ 12.26 (s, 1H), 11.32 (s, 1H), 10.57 (s, 1H), 8.59 (s, 1H), 8.16 (s, 1H), 7.67 (d, J = 7.8 Hz, 1H), 7.51 (d, J = 7.9 Hz, 1H), 7.27 (t, J = 7.9 Hz, 1H), 4.45 (t, J = 6.5 Hz, 2H), 3.99 (q, J = 7.0 Hz, 2H), 3.71 (s, 3H), 2.89 (t, J = 6.5 Hz, 2H), 2.12-2.04 (m, 1H), 1.23 (t, J = 7.0 Hz, 3H), 0.82 (d, J = 5.5 Hz, 4H).

### Example 9

### 4-(3-(3-((6-(Cyclopropylcarboxamido)-3-(ethoxycarbamoyl)pyridazine-4-yl)amino)-2-methoxyphenyl)-1H-1,2,4-triazol-1-yl)butanoic acid (Compound 9)

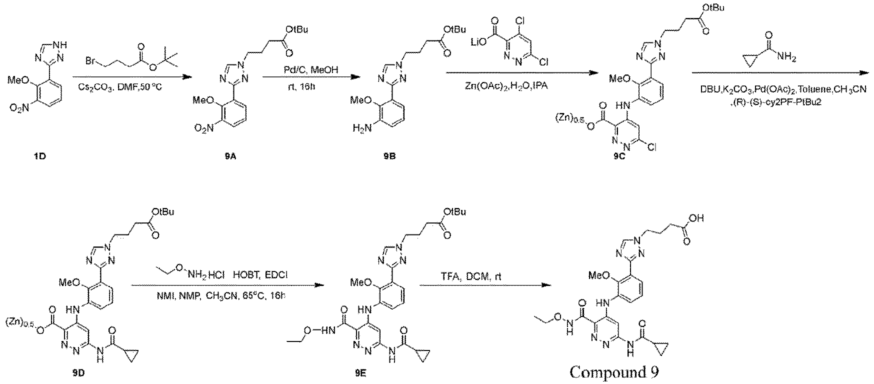

### Step 1: tert-Butyl 4-(3-(2-methoxy-3-nitrophenyl)-1H-1,2,4-triazol-1-yl)butanoate

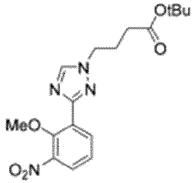

3-(2-Methoxy-3-nitrophenyl)-1*H*-1,2,4-triazole 1 (500 mg, 2.27 mmol) was dissolved in *N,N*-dimethylformamide (10 mL), and tert-butyl 4-bromobutyrate (760 mg, 3.41 mmol) and cesium carbonate (1.48 g, 4.54 mmol) were added. After replacement with nitrogen three times, the reaction solution was stirred in an oil bath at 50°C for 2 hours. The reaction was stopped, and the reaction solution was poured into water (40 mL). The aqueous phase was extracted using ethyl acetate (40 mL × 3), and the merged organic phase was washed with saturated brine (40 mL × 3), dried over anhydrous sodium sulfate and filtered, and then the filtrate was spun in vacuo to remove the solvent to obtain a crude product. The crude product was separated and purified by silica gel column chromatography (petroleum ether:ethyl acetate = 1:1) to obtain the title compound (720 mg, yield: 87.4%, yellow oily matter).

MS (ESI): m/z 363.1 [M+H]+.

### Step 2: tert-Butyl 4-(3-(3-amino-2-methoxyphenyl)-1H-1,2,4-triazol-1-yl)butanoate

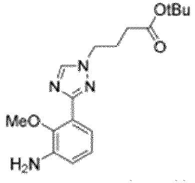

*tert*-Butyl 4-(3-(2-methoxy-3-nitrophenyl)-1*H*-1,2,4-triazol-1-yl)butanoate (720 mg, 1.987 mmol) was dissolved in methanol (12 mL), and a palladium/carbon catalyst (72 mg) was added. After replacement with hydrogen three times, the mixture was allowed to react at room temperature for 16 hours. The reaction was stopped, and filtering was performed to remove the palladium/carbon by suction, and the filtrate was spun in vacuo to remove the solvent to obtain a crude product. The crude product was separated and purified by silica gel column chromatography (dichloromethane:methanol = 15:1) to obtain the title compound (500 mg, yield: 75.8%, colorless and transparent oily matter).

MS (ESI): m/z 333.2 [M+H]+.

### Step 3: ((4-((3-(1-(4-(tert-Butyloxy)-4-oxobutyl)-1H-1,2,4-triazol-3-yl)-2-methoxyphenyl)amino)-6-chloropyridazine-3-carbonyl)oxy)zinc (0.5)

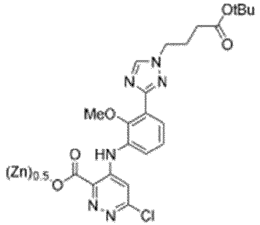

*tert*-Butyl 4-(3-(3-amino-2-methoxyphenyl)-1*H*-1,2,4-triazol-1-yl)butanoate (500 mg, 1.504 mmol) was dissolved in isopropanol (3 mL) and water (21 mL), and lithium 4,6-dichloropyridazine-3-carboxylate (357.4 mg, 1.805 mmol) and zinc acetate (331.2 mg, 1.805 mmol) were added. After replacement with nitrogen three times, the mixture was allowed to react in an oil bath at 65°C for 16 hours. The reaction was stopped, and water (10 mL) was added to the reaction solution. The solution was stirred at room temperature for 1 hour, and then filtered by suction. The filter cake was rinsed using water (10 mL × 2) and tetrahydrofuran (2 mL), and dried to obtain the title compound (620 mg, yield: 79.2%, white solid).

MS (ESI): m/z 489.1 [M+H]+.

### Step 4: ((4-((3-(1-(4-(tert-Butyloxy)-4-oxobutyl)-1H-1,2,4-triazol-3-yl)-2-methoxyphenyl)amino)-6-(cyclopropylcarboxamido)pyridazine-3-carbonyl)oxy)zinc (0.5)

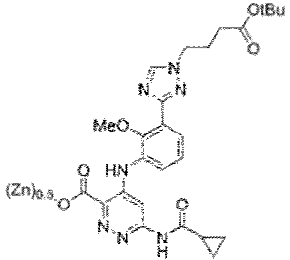

((4-((3-(1-(4-(*tert*-Butyloxy)-4-oxobutyl)-1*H*-1,2,4-triazol-3-yl)-2-methoxyphenyl)amino)-6-chloropyridazine-3-carbonyl)oxy)zinc (0.5) (620 mg, 1.191 mmol) was dissolved in toluene (6 mL) and acetonitrile (3 mL), and cyclopropylcarboxamide (253.4 mg, 2.978 mmol), (*R*)-(-)-1-[(*S*)-2-(dicyclohexylphosphino)ferrocene]ethyl di-*tert*-butylphosphine (132.1 mg, 0.238 mmol), 1,8-diazabicycloundec-7-ene (181.3 mg, 1.191 mmol), potassium carbonate (329.2 mg, 2.382 mmol) and palladium acetate (53.5 mg, 0.238 mmol) were added. After replacement with nitrogen, the mixture was allowed to react in an oil bath at 75°C for 16 hours. The reaction was stopped, the reaction solution was cooled to room temperature, and water (20 mL) and acetic acid (10 mL) were added. The mixed system was washed using petroleum ether (30 mL × 3), the aqueous phase was extracted using dichloromethane (40 mL × 3), and the merged dichloromethane organic phase was washed with saturated brine (40 mL × 3), dried over anhydrous sodium sulfate and filtered, and the filtrate was spun in vacuo to remove the solvent to obtain the title compound (650 mg, yield: 95.9%, brown solid).

MS (ESI): m/z 538.1 [M+H]+.

### Step 5: tert-Butyl 4-(3-((6-(cyclopropylcarboxamido)-3-(ethoxycarbamoyl)pyridazine-4-yl)amino)-2-methoxyphenyl)-1H-1,2,4-triazol-1-yl)butanoate

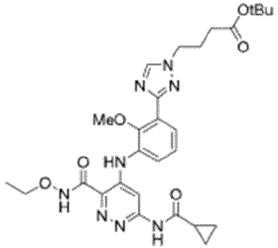

*N*-Methylpyrrolidone (6 mL) and acetonitrile (6 mL) were added to a reaction flask, and ((4-((3-(1-(4-(*tert*-butoxy)-4-oxobutyl)-1*H*-1,2,4-triazol-3-yl)-2-methoxyphenyl)amino)-6-(cyclopropylcarboxamido)pyridazine-3-carbonyl)oxy)zinc (0.5) (650 mg, 1.142 mmol), *O-*ethylhydroxylamine hydrochloride (334.2 mg, 3.426 mmol) and *N*-methylimidazole (281.3 mg, 3.426 mmol) were added. The reaction solution was stirred in an oil bath at 65°C for 15 minutes, and then 1-hydroxybenzotriazole (308.6 mg, 2.284 mmol) and 1-ethyl-(3-dimethylaminopropyl)carbodiimide hydrochloride (437.8 mg, 2.284 mmol) were added. The reaction solution was stirred and allowed to react in an oil bath at 65°C for 16 hours. The reaction was stopped, and the reaction solution was poured into water (40 mL). The aqueous phase was extracted using ethyl acetate (40 mL × 3), and the merged organic phase was washed with saturated brine (40 mL × 3) and dried over anhydrous sodium sulfate. After filtration, the filtrate was spun in vacuo to remove the solvent to obtain the crude title compound (1 g, yellow oily matter), which was directly used for the next step.

MS (ESI): m/z 581.1 [M+H]+.

### Step 6: 4-(3-(3-((6-(Cyclopropylcarboxamido)-3-(ethoxycarbamoyl)pyridazine-4-yl)amino)-2-methoxyphenyl)-1H-1,2,4-triazol-1-yl)butanoic acid (Compound 9)

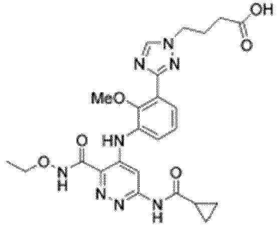

*tert*-Butyl 4-(3-((6-(cyclopropylcarboxamide)-3-(ethoxycarbamoyl)pyridazine-4-yl)amino)-2-methoxyphenyl)-1*H*-1,2,4-triazol-1-yl)butanoate (1 g, 1.722 mmol) was dissolved in dichloromethane (8 mL), and trifluoroacetic acid (2 mL) was added. After replacement with nitrogen three times, the mixture was allowed to react at room temperature for 8 hours. The reaction was stopped, and the reaction solution was spun in vacuo to remove the solvent to obtain a crude product. The crude product was separated and purified by high-performance liquid chromatography (elution system: formic acid, water and acetonitrile) to obtain the title compound (85.41 mg, yield: 9.46%, yellow solid).

MS (ESI): m/z 525.0 [M+H]+.

1H NMR (400 MHz, DMSO-d6) δ 12.25 (s, 1H), 11.31 (s, 1H), 10.56 (s, 1H), 8.61 (s, 1H), 8.16 (s, 1H), 7.68 (dd, J = 7.7, 1.0 Hz, 1H), 7.51 (d, J = 8.0 Hz, 1H), 7.28 (t, J = 7.9 Hz, 1H), 4.28 (t, J = 6.8 Hz, 2H), 4.02-3.97 (m, 2H), 3.72 (s, 3H), 2.29 (t, J = 7.3 Hz, 2H), 2.10-2.04 (m, 3H), 1.23 (t, J = 7.0 Hz, 3H), 0.82 (d, J = 6.0 Hz, 4H).

### Example 10

### 6-(Cyclopropylcarboxamide)-N-ethoxy-4-((3-(1-(2-hydroxyethyl)-1H-1,2,4-triazol-3-yl)-2-methoxyphenyl)amino)pyridazine-3-carboxamide (Compound 10)

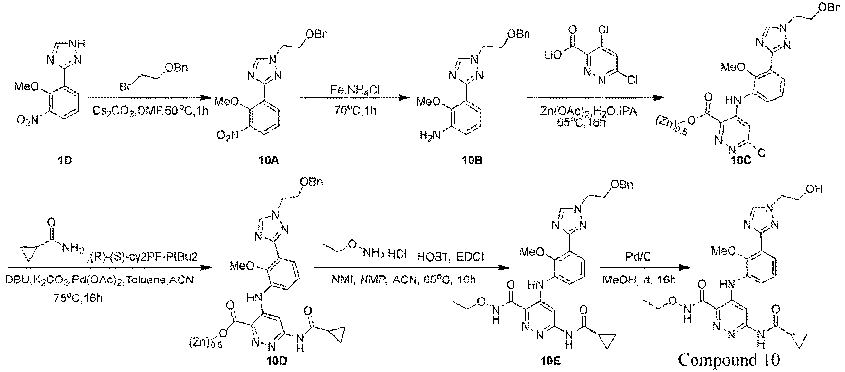

### Step 1: 1-(2-(Benzyloxy)ethyl)-3-(2-methoxy-3-nitrophenyl)-1H-1,2,4-triazole

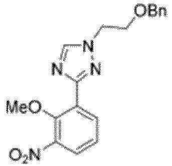

3-(2-Methoxy-3-nitrophenyl)-1H-1,2,4-triazole (1000 mg, 4.542 mmol) was dissolved in ultra-dry N,N-dimethylformamide (10 mL), and cesium carbonate (4440 mg, 13.626 mmol) and ((2-bromoethoxy)methyl)benzene (1465 mg, 6.813 mmol) were added. The mixture was allowed to react at 50°C for 1 hour. The reaction was stopped, and the reaction solution was filtered and then poured into water (20 mL). The aqueous phase was extracted using ethyl acetate (10 mL × 3), and the merged organic phase was washed with saturated brine (10 mL × 3), dried over anhydrous sodium sulfate and filtered, and then the filtrate was spun in vacuo to remove the solvent to obtain a crude product. The crude product was separated and purified by silica gel column chromatography (petroleum ether:ethyl acetate = 3:1) to obtain the title compound (1.5 g, yield: 93.3%, light yellow oily matter)

MS (ESI): m/z 355.5 [M+H]+.

### Step 2: 3-(1-(2-(Benzyloxy)ethyl)-1H-1,2,4-triazol-3-yl)-2-methoxyaniline

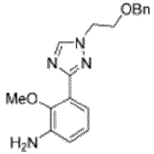

1-(2-(Benzyloxy)ethyl)-3-(2-methoxy-3-nitrophenyl)-1*H*-1,2,4-triazole (1.5 g, 4.233 mmol) was dissolved in isopropanol (10 mL) and water (0.8 mL), and iron powder (233 mg, 21.165 mmol) and ammonium chloride (1132 mg, 21.165) were added. After replacement with nitrogen three times, the mixture was allowed to react at 70°C for 1 hour. The reaction was stopped, and the reaction solution was filtered and then spun dry. A crude product was separated and purified by silica gel column chromatography (petroleum ether:ethyl acetate = 1:5) to obtain the title compound (1.32 g, yield: 96.1%, colorless oily matter).

MS (ESI): m/z 325.0 [M+H]+.

### Step 3: ((4-((3-(1-(2-(Benzyloxy)ethyl)-1H-1,2,4-triazol-3-yl)-2-methoxyphenyl)amino)-6-chloropyridazine-3-carbonyl)oxy)zinc (0.5)

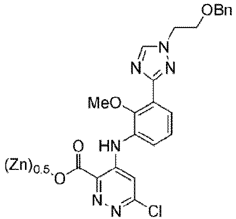

3-(1-(2-(Benzyloxy)ethyl)-1*H*-1,2,4-triazol-3-yl)-2-methoxyaniline (1.2 g, 3.699 mmol) was dissolved in water (35 mL) and isopropanol (5 mL), and lithium 4,6-dichloropyridazine-3-carboxylate (879 mg, 4.439 mmol) and zinc acetate (815 mg, 4.439 mmol) were added. After replacement with nitrogen three times, the mixture was allowed to react in an oil bath at 65°C for 16 hours. The reaction was stopped, and water (40 mL) was added to the reaction solution. The solution was stirred at room temperature for 1 hour, and then filtered by suction. The filter cake was rinsed using water (10 mL × 2) and tetrahydrofuran (1 mL), and dried to obtain the title compound (1.2 g, yield: 63.3%, grey solid).

MS (ESI): m/z 481.1 [M+H]+.

### Step 4: ((4-((3-(1-(2-(Benzyloxy)ethyl)-1H-1,2,4-triazol-3-yl)-2-methoxyphenyl)amino)-6-(cyclopropylcarboxamido)pyridazine-3-carbonyl)oxy)zinc (0.5)

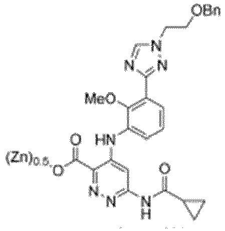

((4-((3-(1-(2-(Benzyloxy)ethyl)-1*H*-1,2,4-triazol-3-yl)-2-methoxyphenyl)amino)-6-chloropyridazine-3-carbonyl)oxy)zinc (0.5) (1.2 g, 2.138 mmol) was dissolved in toluene (24 mL) and acetonitrile (12 mL), and cyclopropylcarboxamide (454 mg, 5.345 mmol), (R)-(-)-1-[(S)-2-(dicyclohexylphosphino)ferrocene]ethyl di-tert-butylphosphine (237 mg, 0.428 mmol), 1,8-diazabicycloundec-7-ene (325 mg, 2.138 mmol), potassium carbonate (590 mg, 4.276 mmol) and palladium acetate (96 mg, 0.428 mmol) were added. After replacement with nitrogen, the mixture was allowed to react in an oil bath at 75°C for 16 hours. The reaction was stopped, and the reaction solution was cooled to room temperature. Water (30 mL) and acetic acid (15 mL) were added, and the mixed system was washed using petroleum ether (50 mL × 2). The aqueous phase was extracted using dichloromethane (10 mL × 3), and the merged dichloromethane organic phase was washed with saturated brine (10 mL × 3), dried over anhydrous sodium sulfate and filtered, and then the filtrate was spun in vacuo to remove the solvent to obtain the title compound (1.8 g, brown oily matter), which was directly used for reaction at the next step.

MS (ESI): m/z 530.0 [M+H]+.

### Step 5: 4-((3-(1-(2-(Benzyloxy)ethyl)-1H-1,2,4-triazol-3-yl)-2-methoxyphenyl)amino)-6-(cyclopropylcarboxamido)-N-ethoxypyridazine-3-carboxamide

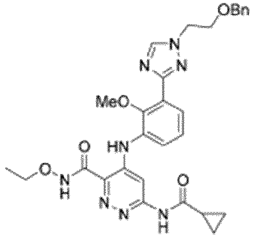

A 25 mL reaction flask was taken, into which *N*-Methylpyrrolidone (2 mL) and acetonitrile (2 mL) were added, and then ((4-((3-(1-(2-(benzyloxy)ethyl)-1*H*-1,2,4-triazol-3-yl)-2-methoxyphenyl)amino)-6-(cyclopropylcarboxamido)pyridazine-3-carbonyl)oxy)zinc (0.5) (400 mg, 0.713 mmol), *O*-ethylhydroxylamine hydrochloride (104 mg, 1.070 mmol) and *N-*methylimidazole (175 mg, 2.139 mmol) were added. The reaction solution was stirred in an oil bath at 65°C for 15 minutes, and then 1-hydroxybenzotriazole (193 mg, 1.426 mmol) and 1-ethyl-(3-dimethylaminopropyl)carbodiimide hydrochloride (273 mg, 1.426 mmol) were added. The reaction solution was stirred in an oil bath at 65°C for 16 hours. The reaction was stopped, and water (20 mL) was added to the reaction solution, followed by extraction using ethyl acetate (10 mL × 3). The organic phases were merged and then washed with saturated brine (10 mL × 3), dried over anhydrous sodium sulfate and filtered, and then the filtrate was spun dry to obtain the title compound (510 mg, brown oily matter), which was directly used for reaction at the next step.

MS (ESI): m/z 573.1 [M+H]+.

### Step 6: 6-(Cyclopropylcarboxamide)-N-ethoxy-4-((3-(1-(2-hydroxyethyl)-1H-1,2,4-triazol-3-yl)-2-methoxyphenyl)amino)pyridazine-3-carboxamide (Compound 10)

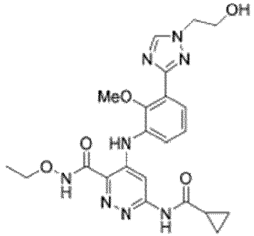

4-((3-(1-(2-(Benzyloxy)ethyl)-1*H*-1,2,4-triazol-3-yl)-2-methoxyphenyl)amino)-6-(cyclopropanecarboxamido)-*N*-ethoxypyridazine-3-carboxamide (510 mg, 0.891 mmol) was dissolved in methanol (20 mL), and palladium carbon (38 mg, 0.356 mmol) was added. After replacement with hydrogen, the mixture was allowed to react at room temperature for 16 hours. The reaction was stopped, and the reaction solution was filtered and then spun dry. A crude product was separated and purified by high-performance liquid chromatography (elution system: formic acid, acetonitrile and water) to obtain the title compound (20.4 mg, yield: 4.7%, white solid).

MS (ESI): m/z 483.0 [M+H]+.

1H NMR (400 MHz, DMSO-d6) δ 8.53 (s, 1H), 8.10 (s, 1H), 7.63 (d, J = 7.7 Hz, 1H), 7.47 (d, J = 8.0 Hz, 1H), 7.24 (t, J = 8.0 Hz, 1H), 5.00 (s, 1H), 4.26 (d, J = 4.9 Hz, 2H), 3.94 (d, J = 6.4 Hz, 2H), 3.78 (brs, 2H), 3.72 (s, 3H), 2.05 (brs, 1H), 1.20 (t, J = 6.7 Hz, 3H), 0.80 (d, J = 5.7 Hz, 4H).

### Example 11

### 6-(Cyclopropylcarboxamido)-N-(2-fluoroethoxy)-4-((3-(1-(2-hydroxyethyl)-1H-1,2,4-triazol-3-yl)-2-methoxyphenyl)amino)pyridazine-3-carboxamide (Compound 11)

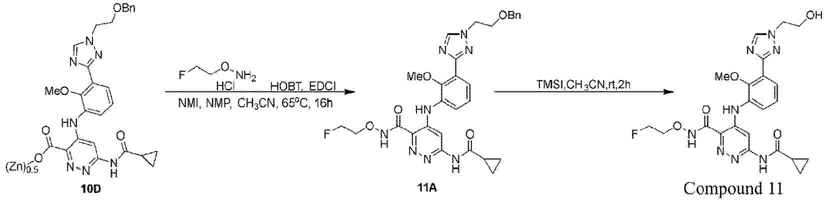

### Step 1: 4-((3-(1-(2-(Benzyloxy)ethyl)-1H-1,2,4-triazol-3-yl)-2-methoxyphenyl)amino)-6-(cyclopropylcarboxamido)-N-(2-fluoroethoxy)pyridazine-3-carboxamide

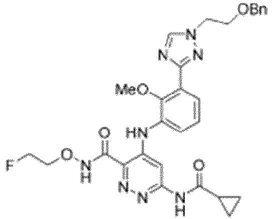

N-Methylpyrrolidone (9 mL) and acetonitrile (9 mL) were stirred at room temperature for ten minutes, and ((4-((3-(1-(2-(benzyloxy)ethyl)-1*H*-1,2,4-triazol-3-yl)-2-methoxyphenyl)amino)-6-(cyclopropylcarboxamido)pyridazine-3-carbonyl)oxy)zinc (0.5) (300 mg, 0.64 mmol), *O*-(2-fluoroethyl)hydroxylamine hydrochloride (110.91 mg, 0.96 mmol) and *N-*methylimidazole (158.45 mg, 1.93 mmol) were added. After replacement with nitrogen, the mixture was allowed to react in an oil bath at 65°C for 15 minutes, and finally 1-hydroxybenzotriazole (172.95 mg, 1.28 mmol) and 1-(3-dimethylaminopropyl)-3-ethylcarbodiimide hydrochloride (245.38 mg, 1.28 mmol) were added. After replacement with nitrogen, the mixture was allowed to react in an oil bath at 65°C for 16 hours. The reaction was stopped, water (20 mL) was added to the reaction solution for quenching, and then extraction was performed using ethyl acetate (30 mL × 3). The merged organic phase was washed with saturated brine, dried over anhydrous sodium sulfate and filtered, and then the filtrate was spun in vacuo to remove the solvent to obtain the title compound (300 mg, yield: 95.1%, brown solid).

MS (ESI): m/z 591.3 [M+H]+.

### Step 2: 6-(Cyclopropylcarboxamido)-N-(2-fluoroethoxy)-4-((3-(1-(2-hydroxyethyl)-1H-1,2,4-triazol-3-yl)-2-methoxyphenyl)amino)pyridazine-3-carboxamide (Compound 11)

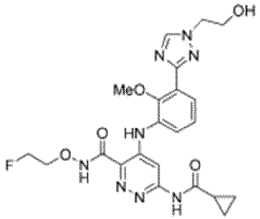

4-((3-(1-(2-(Benzyloxy)ethyl)-1*H*-1,2,4-triazol-3-yl)-2-methoxyphenyl)amino)-6-(cyclopropylcarboxamido)-*N*-(2-fluoroethoxy)pyridazine-3-carboxamide (158 mg, 0.268 mmol) was dissolved in acetonitrile (3 mL), and then trimethylsilyl iodide (267.64 mg, 1.338 mmol) was added. The mixture was allowed to react at room temperature for 4 hours. The reaction was stopped, and the solvent was removed by spinning in vacuo to obtain a crude product. The crude product was separated and purified by reverse phase preparative chromatography (elution system: formic acid, acetonitrile and water) to obtain the title compound (27.91 mg, yield: 20.85%, white solid).

MS (ESI): m/z 501.1 [M+H]+.

1H NMR (400 MHz, DMSO-d6) δ12.46 (s, 1H), 11.34 (s, 1H), 10.51 (s, 1H), 8.56 (s, 1H), 8.15 (s, 1H), 7.70 (dd, J = 7.8, 1.3 Hz, 1H), 7.51 (d, J = 6.7 Hz, 1H), 7.28 (t, J = 7.9 Hz, 1H), 4.77-4.73 (m, 1H), 4.65-4.59 (m, 1H), 4.29 (t, J = 5.3 Hz, 2H), 4.26-4.23 (m, 1H), 4.19-4.14 (m, 1H), 3.80 (t, J = 5.4 Hz, 2H), 3.73 (s, 3H), 2.12-2.05 (m, 1H), 0.85-0.80 (m, 4H).

### Example 12

### 6-(Cyclopropylcarboxamido)-N-(2,2-difluoroethoxy)-4-((3-(1-(2-hydroxyethyl)-1H 1,2,4-triazol-3-yl)-2-methoxyphenyl)amino)pyridazine-3-carboxamide (Compound 12)

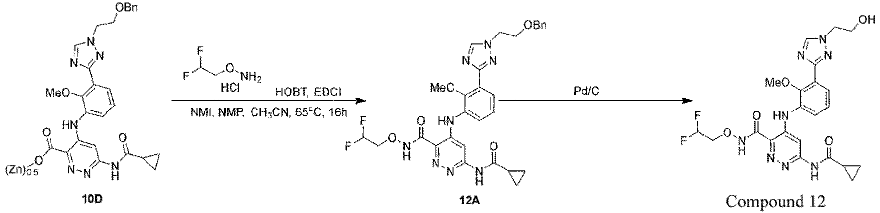

### Step 1: 4-((3-(1-(2-(Benzyloxy)ethyl)-1H-1,2,4-triazol-3-yl)-2-methoxyphenyl)amino)-6-(cyclopropanecarboxamide)-N-(2,2-difluoroethoxy)pyridazine-3-carboxamide

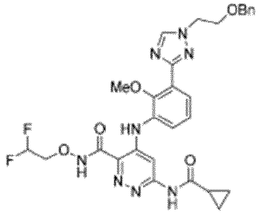

*N*-Methylpyrrolidone (2 mL) and acetonitrile (2 mL) were added to a reaction flask, and then ((4-((3-(1-(2-(benzyloxy)ethyl)-1*H*-1,2,4-triazol-3-yl)-2-methoxyphenyl)amino)-6-(cyclopropylcarboxamido)pyridazine-3-carbonyl)oxy)zinc (0.5) (400 mg, 0.713 mmol), *O*-(2,2-difluoroethyl)hydroxylamine hydrochloride (143 mg, 1.070 mmol) and *N*-methylimidazole (175 mg, 2.139 mmol) were added. The reaction solution was stirred in an oil bath at 65°C for 15 minutes, and then 1-hydroxybenzotriazole (193 mg, 1.426 mmol) and 1-ethyl-(3-dimethylaminopropyl)carbodiimide hydrochloride (273 mg, 1.426 mmol) were added. The reaction solution was stirred and allowed to react in an oil bath at 65°C for 16 hours. The reaction was stopped, and water (20 mL) was added to the reaction solution, followed by extraction using ethyl acetate (10 mL × 3). The organic phases were merged, washed with saturated brine (10 mL × 3), dried over anhydrous sodium sulfate and filtered, and then the filtrate was spun dry to obtain the title compound (290 mg, yield: 66.8%, brown oily matter).

MS (ESI): m/z 609.0 [M+H]+.

### Step 2: 6-(Cyclopropylcarboxamido)-N-(2,2-difluoroethoxy)-4-((3-(1-(2-hydroxyethyl)-1H-1,2,4-triazol-3-yl)-2-methoxyphenyl)amino)pyridazine-3-carboxamide (Compound 12)

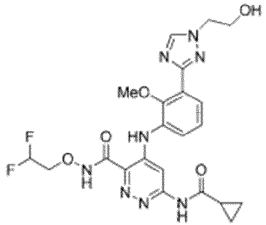

A 50 mL reaction flask was taken, in which 4-((3-(1-(2-(benzyloxy)ethyl)-1H-1,2,4-triazol-3-yl)-2-methoxyphenyl)amino)-6-(cyclopropylcarboxamide)-*N*-(2,2-difluoroethoxy)pyridazine-3-carboxamide (260 mg, 0.427 mmol) was dissolved in methanol (20 mL), and palladium/carbon (18 mg, 0.171 mmol) was added. After replacement with hydrogen, the reaction solution was stirred and allowed to react at room temperature for 40 hours. The reaction was stopped, and the reaction solution was filtered and then spun dry. A crude product was separated and purified by high-performance liquid chromatography (elution system: formic acid, acetonitrile and water) to obtain the title compound (7.65 mg, yield: 3.5%, white solid).

MS (ESI): m/z 519.1 [M+H]+.

1H NMR (400 MHz, DMSO-d6) δ 11.31 (s, 1H), 10.48 (s, 1H), 8.54 (s, 1H), 8.13 (s, 1H), 7.68 (d, J = 7.5 Hz, 1H), 7.50 (d, J = 8.0 Hz, 1H), 7.27 (t, J = 7.9 Hz, 1H), 6.34 (t, J = 54.5 Hz, 1H), 5.00 (s, 1H), 4.29-4.21 (m, 4H), 3.82-3.70 (m, 5H), 2.13-2.01 (m, 1H), 0.81 (d, J = 5.2 Hz, 4H).

### Biological test evaluation

### Test Example A: In vitro TYK2 JH2 enzyme binding assay of the compound of the present invention

Purpose: The purpose of this test example was to use a fluorescence resonance energy transfer (TR-FRET) method to test the binding effect of the compound on a TYK2 JH2 pseudokinase, and then evaluate the affinity of the compound for TYK2 JH2.

### Experimental method:

DMSO-dissolved compounds were used to prepare 10 mM stock solutions, then 200X compounds having different gradient concentrations were prepared in a compound dilution plate, and transferred to an Echo plate. 75 nL of the compounds were transferred from the Echo plate to a 384-well assay plate using an Echo instrument, and 5 µL of 3X TYK2 JH2 pseudokinase (Bioduro), 5 µL of 3X Tb antibody (Cisbio) and 5 µL of 3X TRACER (Bioduro) were added to the 384-well assay plate, centrifuged for 30 seconds, and incubated at room temperature for 60 minutes. A 495 nm/520 nm fluorescence signal ratio was read by an Envision microplate reader (PerkinElmer), and data was analyzed using XL-Fit software to calculate the IC₅₀ of the compound.

### Experimental results:

| Compound number | TYK2 JH2 binding assay (IC₅₀, nM) |
|---|---|
| Compound 1 | 0.21 |
| Compound 2 | 0.19 |
| Compound 4 | 0.18 |
| Compound 5 | 0.26 |
| Compound 7 | 0.29 |
| Compound 8 | 0.29 |
| Compound 9 | 0.42 |
| Compound 10 | 0.28 |
| BMS-986165 | 0.25 |

It can be seen from the experimental data that the compound of the present invention had a good binding effect on TYK2 JH2 pseudokinase.

### Test Example B: Determination of the effects of the compound of the present invention on IL-2-induced STAT5 phosphorylation (JAK1/3) in CD3+ cell subpopulations in human PBMC

Purpose: The purpose of this test case was to test the inhibitory effect of the compound on IL-2-induced STAT5 phosphorylation using protein phosphorylation flow cytometric analysis technology.

### Experimental method:

Human PBMC cells were pre-incubated with the compounds, STAT5 was induced to phosphorylate under appropriate stimulation conditions, and corresponding cell subpopulations and targets were stained. Flow cytometry was used to read cell data and analyze the strength of phosphorylated antibody signals under different compound concentrations. PBMCs were resuspended and dispensed into a 96-well plate, 62.5 µL/well. 3.5 µL of 20X compound working solutions were added and incubated at 37°C for 30 minutes. 5 µL of PE mouse anti-human CD3 (BD) was added and incubated for 30 min at 37°C in the dark. 4 µL of 20X IL-2 (R&D) was added to each well and incubated at 37°C in the dark for 20 minutes. All cells in the 96-well plate were transferred to a deep-well plate, 400 µL of a fixation working solution (Biolegend) was added to each well, and incubated at room temperature in the dark for 20 minutes. The cells were washed twice with PBS, and 400 µL of Perm Buffer III (BD) was added and incubated at 4°C in the dark for 40 minutes. The cells were washed twice with PBS, and 100 µL of a STATS pY694 antibody working solution (BD) was added and incubated at room temperature for 40 minutes. After washing once with PBS, the cells were resuspended in 200 µL of a staining buffer and transferred to a sample plate. The antibody fluorescence intensity was analyzed using FlowJo software, and the IC₅₀ of the compound was calculated using XL-Fit software.

### Experimental results:

| | |
|---|---|
| Compound number | IL-2-induced CD3+ pSTAT5 (JAK1/3) in hPBMC (IC₅₀, µM) |
| Compound 1 | >30 |
| Compound 2 | >30 |
| BMS-986165 | 0.409 |

It can be seen from the experimental data results that the compound of the present invention had lower inhibitory activity on the JAK1/3 signaling pathway and had better selectivity.

### Test Example C: Determination of JAK1 JH1/JAK2 JH1/JAK3 JH1/TYK2 JH1 kinase inhibition by the compound of the present invention

Purpose: The purpose of this test example was to examine the inhibitory effect of the compound on the kinase activity of JAK1 JH1/JAK2 JH1/JAK3 JH1/TYK2 JH1 using homogeneous time-resolved fluorescence resonance energy transfer (HTRF) technology.

### Experimental method:

100 nL/well of compound working solutions were transferred to a 384-well assay plate using an automated micropipetting system, 5 µL/well of 2X JAK1 JH1/JAK2 JH1/JAK3 JH1/TYK2 JH1 kinase solutions or assay buffer were added, uniformly mixed by centrifugation, and incubated at the room temperature 25°C for 15 minutes. After the completion of incubation, 5 µL of a mixed solution containing a 2X TK-SUB-biotin substrate and ATP was added to each well, uniformly mixed by centrifugation, and incubated at 25°C for 45 minutes (JAK1/JAK2) or 60 minutes (JAK3/TYK2). After completion of incubation, 10 µL of an assay mixture (TK Antibody-EU and Streptavidin-XL mixture) was added to each well, uniformly mixed by centrifugation, and incubated at 25°C for 60 minutes (JAK1/JAK2) or 120 minutes (JAK3/TYK2). After completion of incubation, the solutions were transferred to a 4°C freezer for incubation overnight, and fluorescence values were read on an Envision 2104 Multilabel Reader, and the IC₅₀ of the compound was calculated using XL-Fit software.

### Experimental results:

| Compound number | Inhibition on kinase activity (IC₅₀, µM) | | | |
|---|---|---|---|---|
| | JAKI JHI | JAK2 JHI | JAK3 JHI | TYK2 JHI |
| Compound 1 | >10 | >10 | >10 | >10 |
| Compound 2 | >10 | >10 | >10 | >10 |
| BMS-986165 | 1.58 | 3.62 | 1.27 | >10 |

It can be seen from the experimental data results that the compound of the present invention had lower inhibitory activity on JAK1 JH1/JAK2 JH1/JAK3 JH1 and had better selectivity.

## Claims

1. A compound represented by general formula (I), or a pharmaceutically acceptable salt, enantiomer, diastereomer, racemate, solvate, hydrate, polymorph, prodrug or isotopic variant thereof, and a mixture thereof: 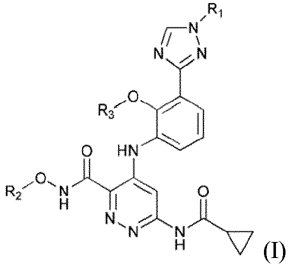 wherein
R₁ is selected from H, a C₁₋₆ haloalkyl, a C₁₋₆ alkylene-ORₐ, a C₁₋₆ alkylene-SRₐ, a C₁₋₆ alkylene-NR_{b}R_{c}, a C₁₋₆ alkylene-C(O)Rₐ, a C₁₋₆ alkylene-C(O)ORₐ, a C₁₋₆ alkylene-C(O)NR_{b}R_{c}, a C₁₋₆ alkylene-OC(O)Rₐ, a C₁₋₆ alkylene-NR_{b}C(O)Rₐ, a C₁₋₆ alkylene-S(O)ₘRₐ, a C₁₋₆ alkyleneS(O)ₘNR_{b}R_{c} or a C₁₋₆ alkylene-NR_{b}S(O)ₘRₐ, and the group is optionally substituted with one or more deuteriums until fully deuterated;
R₂ is selected from a C₁₋₆ alkyl, a C₁₋₆ haloalkyl, a C₂₋₆ alkenyl, a C₂₋₆ alkynyl, a C₃₋₇ cycloalkyl, a 3- to 7-membered heterocyclyl, a C₆₋₁₀ aryl or a 5- to 10-membered heteroaryl, and the group is optionally substituted with one or more deuteriums until fully deuterated;
R₃ is selected from H, a C₁₋₆ alkyl or a C₁₋₆ haloalkyl, and the group is optionally substituted with one or more deuteriums until fully deuterated;
each group of R₁, R₂ and R₃ is optionally substituted with 1, 2, 3 or 4 R;
wherein Rₐ, R_{b} and R_{c} are independently selected from H, a C₁₋₆ alkyl, a C₁₋₆ haloalkyl, a C₂₋₆ alkenyl, a C₂₋₆ alkynyl, a C₃₋₇ cycloalkyl, a 3- to 7-membered heterocyclyl, a C₆₋₁₀ aryl or a 5-to 10-membered heteroaryl;
m is 1 or 2; and
R is selected from H, D, a halogen, CN, a C₁₋₆ alkyl or a C₁₋₆ haloalkyl.

2. The compound, or the pharmaceutically acceptable salt, enantiomer, diastereomer, racemate, solvate, hydrate, polymorph, prodrug or isotopic variant thereof, and the mixture thereof according to claim 1, wherein R₁ is selected from H, a C₁₋₆ haloalkyl, a C₁₋₆ alkylene-ORₐ, a C₁₋₆ alkylene-SRₐ, a C₁₋₆ alkylene-NR_{d}R_{c}, a C₁₋₆ alkylene-C(O)Rₐ, a C₁₋₆ alkylene-C(O)ORₐ, a C₁₋₆ alkylene-C(O)NR_{b}R_{c}, a C₁₋₆ alkylene-OC(O)Rₐ or a C₁₋₆ alkylene-NR_{b}C(O)Rₐ, and the group is optionally substituted with one or more deuteriums until fully deuterated; preferably, R₁ is selected from H, a C₁₋₆ haloalkyl, a C₁₋₆ alkylene-ORₐ, a C₁₋₆ alkylene-SRₐ, a C₁₋₆ alkylene-NR_{d}R_{c}, a C₁₋₆ alkylene-C(O)ORₐ or a C₁₋₆ alkylene-C(O)NR_{b}R_{c}, and the group is optionally substituted with one or more deuteriums until fully deuterated; preferably, R₁ is selected from H, a C₁₋₆ haloalkyl, a C₁₋₆ alkylene-OH, a C₁₋₆ alkylene-SH, a C₁₋₆ alkylene-NH₂, a C₁₋₆ alkylene-C(O)OH or a C₁₋₆ alkylene-C(O)NH₂, and the group is optionally substituted with one or more deuteriums until fully deuterated; preferably, R₁ is selected from H or a C₁₋₆ haloalkyl, and the group is optionally substituted with one or more deuteriums until fully deuterated; and preferably, R₁ is a C₁₋₆ alkylene-ORₐ, wherein Rₐ is selected from a C₁₋₆ alkyl or a C₁₋₆ haloalkyl.

3. The compound, or the pharmaceutically acceptable salt, enantiomer, diastereomer, racemate, solvate, hydrate, polymorph, prodrug or isotopic variant thereof, and the mixture thereof according to claim 1 or 2, wherein R₂ is selected from a C₁₋₆ alkyl, a C₁₋₆ haloalkyl, a C₂₋₆ alkenyl, a C₂₋₆ alkynyl, a C₃₋₇ cycloalkyl or a 3- to 7-membered heterocyclyl, and the group is optionally substituted with one or more deuteriums until fully deuterated; preferably, R₂ is selected from a C₁₋₆ alkyl, a C₁₋₆ haloalkyl, a C₃₋₇ cycloalkyl or a 3- to 7-membered heterocyclyl, and the group is optionally substituted with one or more deuteriums until fully deuterated; and preferably, R₂ is selected from a C₁₋₆ alkyl or a C₃₋₇ cycloalkyl, and the group is optionally substituted with one or more deuteriums until fully deuterated.

4. The compound, or the pharmaceutically acceptable salt, enantiomer, diastereomer, racemate, solvate, hydrate, polymorph, prodrug or isotopic variant thereof, and the mixture thereof according to any one of claims 1 to 3, wherein R₃ is selected from a C₁₋₆ alkyl or a C₁₋₆ haloalkyl, and the group is optionally substituted with one or more deuteriums until fully deuterated; and preferably, R₃ is methyl, which is optionally substituted with one or more deuteriums until fully deuterated.

5. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is selected from H, a C₁₋₆ haloalkyl, a C₁₋₆ alkylene-ORₐ, a C₁₋₆ alkylene-SRₐ or a C₁₋₆ alkylene-C(O)ORₐ, and the group is optionally substituted with one or more deuteriums until fully deuterated;
R₂ is selected from a C₁₋₆ alkyl, a C₁₋₆ haloalkyl, a C₃₋₇ cycloalkyl or a 3- to 7-membered heterocyclyl, and the group is optionally substituted with one or more deuteriums until fully deuterated;
R₃ is selected from H, a C₁₋₆ alkyl or a C₁₋₆ haloalkyl, and the group is optionally substituted with one or more deuteriums until fully deuterated;
each group of R₁, R₂ and R₃ is optionally substituted with 1, 2, 3 or 4 R;
wherein Rₐ is selected from H, a C₁₋₆ alkyl or a C₁₋₆ haloalkyl; and
R is selected from H, D, a halogen, CN, a C₁₋₆ alkyl or a C₁₋₆ haloalkyl.

6. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is selected from H, a C₁₋₄ haloalkyl, a C₁₋₄ alkylene-ORₐ or a C₁₋₄ alkylene-C(O)OH, and the group is optionally substituted with one or more deuteriums until fully deuterated;
R₂ is selected from a C₁₋₄ alkyl, a C₁₋₄ haloalkyl or a C₃₋₇ cycloalkyl, and the group is optionally substituted with one or more deuteriums until fully deuterated; and
R₃ is selected from a C₁₋₄ alkyl or a C₁₋₄ haloalkyl, and the group is optionally substituted with one or more deuteriums until fully deuterated;
wherein Rₐ is selected from H, a C₁₋₄ alkyl or a C₁₋₄ haloalkyl.

7. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is a C₁₋₄ alkylene-ORₐ, which is optionally substituted with one or more deuteriums until fully deuterated;
R₂ is selected from a C₁₋₄ alkyl, a C₁₋₄ haloalkyl or a C₃₋₇ cycloalkyl, and the group is optionally substituted with one or more deuteriums until fully deuterated; preferably a C₁₋₄ alkyl, which is optionally substituted with one or more deuteriums until fully deuterated; and
R₃ is selected from a C₁₋₄ alkyl or a C₁₋₄ haloalkyl, and the group is optionally substituted with one or more deuteriums until fully deuterated;
wherein Rₐ is selected from a C₁₋₄ alkyl or a C₁₋₄ haloalkyl.

8. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is selected from H, a C₁₋₄ haloalkyl, a C₁₋₄ alkylene-OH or a C₁₋₄ alkylene-C(O)OH, and the group is optionally substituted with one or more deuteriums until fully deuterated;
R₂ is selected from a C₁₋₄ alkyl, a C₁₋₄ haloalkyl or a C₃₋₇ cycloalkyl, and the group is optionally substituted with one or more deuteriums until fully deuterated; preferably a C₁₋₄ alkyl, which is optionally substituted with one or more deuteriums until fully deuterated; and
R₃ is selected from a C₁₋₄ alkyl or a C₁₋₄ haloalkyl, and the group is optionally substituted with one or more deuteriums until fully deuterated.

9. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is selected from H, a C₁₋₆ alkylene-ORₐ and a C₁₋₆ alkylene-SRₐ, and the group is optionally substituted with one or more deuteriums until fully deuterated;
R₂ is selected from a C₁₋₆ alkyl and a C₁₋₆ haloalkyl, and the group is optionally substituted with one or more deuteriums until fully deuterated;
R₃ is selected from H, a C₁₋₆ alkyl or a C₁₋₆ haloalkyl, and the group is optionally substituted with one or more deuteriums until fully deuterated;
each group of R₁, R₂ and R₃ is optionally substituted with 1, 2, 3 or 4 R;
wherein Rₐ is selected from H, a C₁₋₆ alkyl and a C₁₋₆ haloalkyl; and
R is selected from H, D, a halogen, CN, a C₁₋₆ alkyl or a C₁₋₆ haloalkyl.

10. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is selected from H and a C₁₋₆ alkylene-ORₐ, and the group is optionally substituted with one or more deuterium groups until fully deuterated;
R₂ is selected from a C₁₋₆ alkyl and a C₁₋₆ haloalkyl; and
R₃ is a C₁₋₆ alkyl, and the group is optionally substituted with one or more deuteriums until fully deuterated;
wherein Rₐ is selected from H and a C₁₋₆ alkyl.

11. The compound, or the pharmaceutically acceptable salt, enantiomer, diastereomer, racemate, solvate, hydrate, polymorph, prodrug or isotopic variant thereof, and the mixture thereof according to claim 1, wherein
R₁ is selected from H and a C₁₋₄ alkylene-ORₐ, and the group is optionally substituted with one or more deuterium groups until fully deuterated;
R₂ is a C₁₋₄ alkyl, and the group is optionally substituted with one or more deuteriums until fully deuterated; and
R₃ is a C₁₋₄ alkyl, and the group is optionally substituted with one or more deuteriums until fully deuterated;
wherein Rₐ is a C₁₋₄ alkyl.

12. The compound, or the pharmaceutically acceptable salt, enantiomer, diastereomer, racemate, solvate, hydrate, polymorph, prodrug or isotopic variant thereof, and the mixture thereof according to claim 1, wherein the compound is selected from:

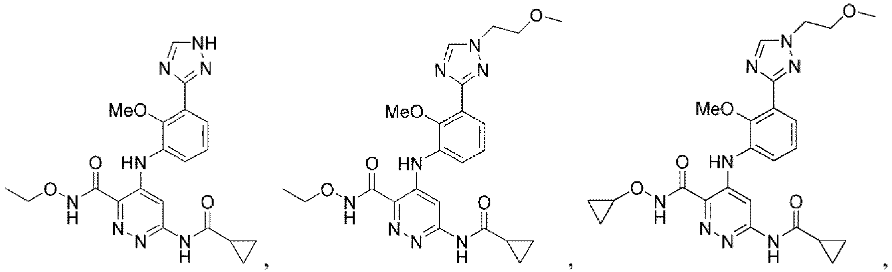

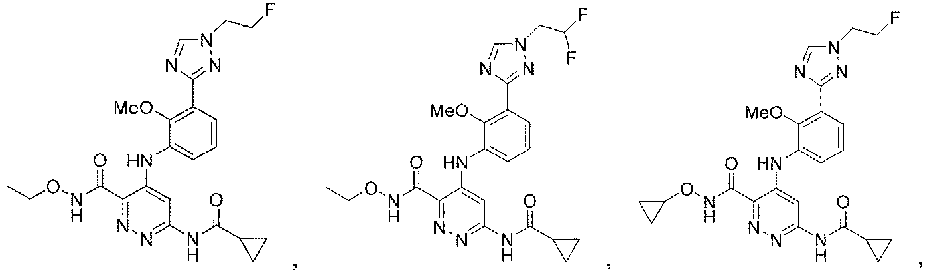

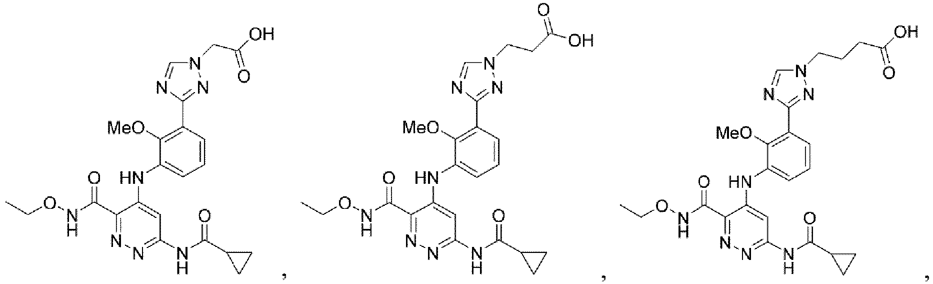

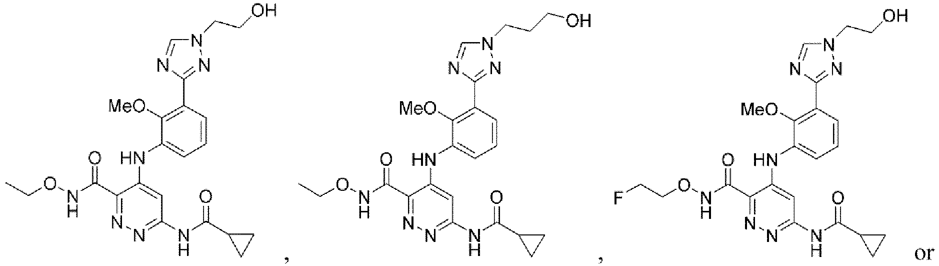

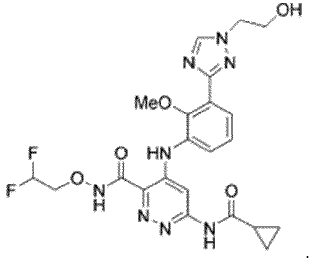

13. A pharmaceutical composition, comprising the compound, or the pharmaceutically acceptable salt, enantiomer, diastereomer, racemate, solvate, hydrate, polymorph, prodrug or isotopic variant thereof, and the mixture thereof according to any one of claims 1 to 12, and a pharmaceutically acceptable excipient; wherein preferably, the pharmaceutical composition further comprises other therapeutic agents.

14. A use of the compound or the pharmaceutically acceptable salt, enantiomer, diastereomer, racemate, solvate, hydrate, polymorph, prodrug or isotopic variant thereof, and the mixture thereof according to any one of claims 1 to 12 in the preparation of a medicament for treating and/or preventing a TYK2 kinase-mediated disease.

15. A use of the compound or the pharmaceutically acceptable salt, enantiomer, diastereomer, racemate, solvate, hydrate, polymorph, prodrug or isotopic variant thereof, and the mixture thereof according to any one of claims 1 to 12, or the pharmaceutical composition according to claim 13 for treating and/or preventing a TYK2 kinase-mediated disease.

16. A method for treating and/or preventing a TYK2 kinase-mediated disease in a subject, comprising administering to the subject the compound or the pharmaceutically acceptable salt, enantiomer, diastereomer, racemate, solvate, hydrate, polymorph, prodrug or isotopic variant thereof, and the mixture thereof according to any one of claims 1 to 12, or the pharmaceutical composition according to claim 13.

17. The use according to claim 14 or the use of the compound or pharmaceutical composition according to claim 15 or the method according to claim 16, wherein the TYK2 kinase-mediated disease is selected from an autoimmune disease, a skin disease, an allergic disease, organ rejection, cancer, dry eye disease, myelofibrosis and polycythemia vera; preferably, the autoimmune disease is lupus, multiple sclerosis, rheumatoid arthritis, juvenile arthritis, psoriasis, ulcerative colitis, Crohn's disease or autoimmune thyroid disease; the skin disease is psoriasis, a rash or atopic dermatitis; the allergic disease is asthma or rhinitis; the organ transplant rejection is allograft rejection or graft-versus-host disease; and the cancer is renal cancer, liver cancer, pancreatic cancer, gastric cancer, breast cancer, prostate cancer, head and neck cancer, thyroid cancer, lung cancer, glioblastoma, melanoma, lymphoma or leukemia.

18. The use according to claim 14 or the use of the compound or pharmaceutical composition according to claim 15 or the method according to claim 16, wherein the TYK2 kinase-mediated disease is selected from rheumatoid arthritis, psoriasis, ulcerative colitis and Crohn's disease.
